# EUROPEAN PATENT SPECIFICATION

(11) **EP 2 196 534 B1**
(45) Date of publication and mention of the grant of the patent: **24.08.2016**
(21) Application number: 08831819.1
(22) Date of filing: 19.09.2008
(51) Int. Cl.: C12N 1/21, C12N 15/09, C12P 13/00

(54) **A RECOMBINANT MICROORGANISM AND METHOD FOR PRODUCING POLY-GAMMA-GLUTAMIC ACID**
REKOMBINANTER MIKROORGANISMUS UND VERFAHREN ZUR HERSTELLUNG VON POLY-GAMMA-GLUTAMINSÄURE
MICROORGANISME RECOMBINANT ET PROCÉDÉ DE PRODUCTION D'ACIDE POLY-GAMMA-GLUTAMIQUE

(30) Priority: 20.09.2007 JP 2007244023
(43) Date of publication of application: 16.06.2010
(73) Proprietor: Kao Corporation, Chuo-Ku Tokyo 103-8210 (JP)
(72) Inventor: SAWADA, Kazuhisa, Haga-gun Tochigi 321-3497 (JP); HAGIHARA, Hiroshi, Haga-gun Tochigi 321-3497 (JP)
(74) Representative: Vossius & Partner Patentanwälte Rechtsanwälte mbB
(86) International application number: PCT/JP2008/067023
(87) International publication number: WO 2009/038194

(56) References cited:
- JP-A- 2001 017 182
- ASHIUCHI M ET AL: "Biochemistry and molecular genetics of poly-gamma-glutamate synthesis.", APPLIED MICROBIOLOGY AND BIOTECHNOLOGY JUN 2002 LNKD- PUBMED:12073126, vol. 59, no. 1, June 2002 (2002-06), pages 9-14, XP009077820, ISSN: 0175-7598
- ASHIUCHI, M. ET AL.: 'Genetically engineered poly-gamma-glutamate producer from Bacillus subtilis ISW1214.' BIOSCI. BIOTECHNOL. BIOCHEM. vol. 70, no. 7, July 2006, pages 1794 - 1797, XP008132383
- ASHIUCHI, M. ET AL.: 'Enzymatic synthesis of high-molecular-mass poly-gamma-glutamate and regulation of its stereochemistry.' APPL. ENVIRON. MICROBIOL. vol. 70, no. 7, July 2004, pages 4249 - 4255, XP002339209
- CANDELA, T. ET AL.: 'Poly-gamma-glutamate in bacteria.' MOL. MICROBIOL. vol. 60, no. 5, June 2006, pages 1091 - 1098, XP002468650
- URUSHIBATA, Y. ET AL.: 'Characterization of the Bacillus subtilis ywsC gene, involved in gamma-polyglutamic acid production.' J. BACTERIOL. vol. 184, no. 2, January 2002, pages 337 - 343, XP002339775
- ASHIUCHI, M. ET AL.: 'Physiological and biochemical characteristics of poly gamma- glutamate synthetase complex of Bacillus subtilis.' EUR. J. BIOCHEM. vol. 268, October 2001, pages 5321 - 5328, XP008132387
- ASHIUCHI, M. ET AL.: 'A poly-gamma-glutamate synthetic system of Bacillus subtilis IFO 3336: gene cloning and biochemical analysis of poly-gamma-glutamate produced by Escherichia coli clone cells.' BIOCHEM. BIOPHYS. RES. COMMUN. vol. 263, September 1999, pages 6 - 12, XP002986618
- HARUO MISONO: 'Biopolymer, Poly-gamma-glutamate no Seigosei Kiko' JAPAN SOCIETY FOR BIOSCIENCE, BIOTECHNOLOGY, AND AGROCHEMISTRY CHUSHIKOKU SHIBU DAI 6 KAI KOENKAI KOEN YOSHISHU 31 May 2003, page 10

## Description

### TECHNICAL FIELD

The present invention relates to a recombinant microorganism having poly-γ-glutamic acid-producing ability and a method of producing poly-γ-glutamic acid by employing the recombinant microorganism.

### BACKGROUND ART

Poly-γ-glutamic acid is a polymeric compound of glutamic acid, in which the γ-carboxyl and α-amino groups thereof are bound to each other, forming peptide bonds. The poly-γ-glutamic acid is also called γ-polyglutamic acid. The poly-γ-glutamic acid is known as a viscous substance produced by *Bacillus subtilis* var. *natto.* This compound has been attracting attention as a new polymeric raw material because of its various properties. In the description below, "poly-γ-glutamic acid" will be also referred to simply as "PGA".

Examples of PGA-producing microorganisms include some of *Bacillus* microbes including *Bacillus subtilis* var. *natto* and the allied species *(Bacillus subtilis* var. *chungkookjang, Bacillus licheniformis, Bacillus megaterium, Bacillus anthracis,* and *Bacillus halodurans), Natrialba aegyptiaca,* and *Hydra* (see, for example, Non-Patent Document 1). Among the microorganisms above, the PGA-producing *Bacillus subtilis* var. *natto* is taxonomically classified into a subspecies of PGA-nonproducing *Bacillus subtilis,* and it is known that any strain of the microorganism has *pgsB, pgsC* and *pgsA* genes coding PGA-producing enzymes at sites downstream of the same transcription initiation regulatory region and the same translation initiation regulatory region, forming a cluster structure (operon) (see, for example, Non-Patent Documents 3, 6 and 8). In addition, the PGA production-controlling mechanism of *Bacillus subtilis* var. *natto* is considered to be regulated in a complicated way in which various intercellular control factors are involved, similarly to the mechanism of *Bacillus subtilis* acquiring transforming ability (see, for example, Non-Patent Documents 9 and 10). It is possible to improve the productivity of the PGA by breeding of the PGA-producing microorganism as a method of producing PGA. However, such a method demands a great amount of labor needed for elimination of the strict regulation mechanism in wild strain, elimination of complicated nutritional requirements in wild strain, or optimized stabilized productivity of the production process. Thus, it is not considered to be an efficient method for producing PGA. For that reason, use of gene recombination technology is studied as an efficient method replacing the conventional breeding of wild strains. For example, as a method of producing PGA by the gene recombination technology, it was disclosed that a *Bacillus subtilis* recombinant containing genes introduced with plasmid (*Bacillus subtilis* ISW1214 strain) has a productivity of approximately 9.0 g/L/5 days (see Non-Patent Document 2) and that a *Escherichia coli* recombinant containing genes introduced with plasmid has a productivity of approximately 4 g/L/1.5 days (see Non-Patent Document 7) or of 2.5 mg/40 mg-drying microbe (see Non-Patent Document 6). However, the productivity by these producing methods disclosed above is still considered insufficient.

PGA is shown to be ribosome-independently produced by a membrane-binding enzyme system called PgsBCA in *Bacillus subtilis* var. *natto* (see, for example, Non-Patent Document 4). Among the enzymes in PGA synthetase system PgsBCA, PgsB (which is also referred to as YwsC) is known to be involved in amide ligase reaction, as it acts on the γ-carboxyl group of glutamic acid (see, e.g., Non-Patent Documents 3 and 5). On the other hand, according to Non-Patent Document 3, PgsC (which is also referred to as YwtA) is considered to be a membrane-binding protein involved in extracellular excretion of PGA. However, according to Non-Patent Documents 4 and 5, PgsC is said to be involved in ligation reaction of glutamic acids in combination with PgsB. In this situation, the function of PgsC remains unresolved even now.

Further, Non-Patent Document 2 discloses the results obtained by studying the PGA productivity of recombinant *Bacillus subtilis* strains that are deficient of the *pgsB, pgsC* and *pgsA* genes on chromosome but contain an introduced vector having one gene selected from *pgsB, pgsC* and *pgsA* genes or a vector having multiple genes selected therefrom in combination. Non-Patent Document 2 concludes that expression of all *pgsB, pgsC* and *pgsA* genes are needed for production of PGA. Alternatively, in Non-Patent Document 3, a strain deficient in *pgsB* gene (which is also referred to as *ywsC* gene), a strain deficient in *pgsC* gene (which is also referred to as *ywtA* gene) and a strain deficient in *pgsA* gene (which is also referred to as *ywtB* gene) of *Bacillus subtilis* var. *natto* (IFO16449 strain) having an ability of producing PGA were prepared and the ability of producing PGA of the deficient strains was examined. Non-Patent Document 3 concludes that the *pgsB* gene and the *pgsC* gene are essential in PGA production in *Bacillus subtilis* var. *natto* having an ability of producing PGA and the *pgsA* gene is needed for further improvement in productivity of PGA.

Patent Document 1 also discloses that *Escherichia coli* strain inherently having no ability of producing PGA gains the ability of producing PGA, when transformed with a vector containing *pgsB* gene, *pgsC* gene and *pgsA* gene in that order. Non-Patent Document 6 discloses that, in a recombinant *Escherichia coli* strain, PGA was produced under the condition when a *pgsBCA* gene was introduced, but no PGA was produced under the condition when a *pgsBC* gene was introduced.

Patent Documents 2 to 5 disclose that PGAs having an average molecular weight of several thousands to 2,000,000 were obtained when a PGA-producing microorganism such as *Bacillus subtilis* var. *natto* was cultured in various nutrition salt media. Patent Document 6 discloses that a strain of *Bacillus subtilis* var. *natto* produced PGA having a molecular weight of 3,000,000 or more under application of external stress.

Non-Patent Document 11 and Patent Documents 7 and 8 disclosed that it is possible to produce PGA high in optical purity by microorganisms. Patent Document 7 discloses that it is possible to produce PGA having a L-glutamic acid content of 90% or more at a productivity of approximately 10 g/L/5 days by employing a *Bacillus megaterium* strain. Patent Document 8 discloses that a *Natrialba aegyptiaca* mutant strain produces poly-γ-L-glutamic acid having a molecular weight of 1,300,000 or more at a productivity of approximately 5 g/L/6 days.

[Non-Patent Document 1] Ashiuchi, M., et al.: Appl. Microbiol. Biotechnol., 59, pp. 9-14 (2002)
[Non-Patent Document 2] Ashiuchi, M., et al.: Biosci. Biotechnol. Biochem., 70, pp. 1794-1797 (2006)
[Non-Patent Document 3] Urushibata, Y., et al.: J. Bacteriol., 184, pp. 337-343 (2002)
[Non-Patent Document 4] Makoto ASHIUCHI, et al.: Mirai Zairyo (Materials for the Future), 3, pp. 44-50 (2003)
[Non-Patent Document 5] Ashiuchi, M., et al.: Eur. J. Biochem., 268, pp. 5321-5328 (2001)
[Non-Patent Document 6] Ashiuchi, M., et al.: Biochem. Biophys. Res. Commun., 263, pp. 6-12 (1999)
[Non-Patent Document 7] Jiang, H., et al.: Biotechnol. Lett., 28, pp. 1241-1246 (2006)
[Non-Patent Document 8] Presecan, E., et al.: Microbiology, 143, pp. 3313-3328 (1997)
[Non-Patent Document 9] Itaya, M., et al.: Biosci. Biotechnol. Biochem., 63, pp. 2034-2037 (1999)
[Non-Patent Document 10] Tadayuki IMANAKA, et al.: Biseibutsu Riyou no Daitenkai (Great Development of Use of Microorganisms), first chapter, fourth section, pp. 657-663 (2002)
[Non-Patent Document 11] Shimizu, K., et al.: Appl. Environ. Microbiol., 73, pp. 2378-2379 (2007)
[Patent Document 1] JP-A-2001-17182 ("JP-A" means unexamined published Japanese patent application)
[Patent Document 2] JP-B-43-24472 ("JP-B" means examined Japanese patent publication)
[Patent Document 3] JP-A-1-174397
[Patent Document 4] JP-A-3-47087
[Patent Document 5] Japanese Patent No. 3081901
[Patent Document 6] JP-A-2006-42617
[Patent Document 7] JP-A-2007-228957
[Patent Document 8] JP-A-2007-314434

### DISCLOSURE OF INVENTION

However, no method of producing PGA at high yield was disclosed in Non-Patent Documents 1 to 3 and 7 and Patent Document 1 described above, and unfavorably, it was impossible to produce PGA efficiently by traditional methods. There was also no report on adjustment of the molecular weight of PGA by genetic engineering method.

The present invention, which was made under the circumstances above, resides in to provide: a recombinant microorganism capable of producing PGA efficiency; a method of producing PGA by employing the microorganism; a method of improving PGA productivity by introduction of genes; a method of adjusting molecular weight of PGA; and a method of adjusting optical purity of PGA.

The present invention relates to the embodiments as defined in the claims. Thus, it relates to the following items:
1. A recombinant microorganism having ability of producing poly-γ-glutamic acid, wherein, the recombinant microorganism is being prepared by introducing a *Bacillus subtilis pgsB* gene, and a *Bacillus subtilis pgsC* gene into a host microorganism, wherein no *Bacillus subtilis pgsA* gene or a gene having a nucleotide sequence with a genetic identity to the nucleotide sequence set forth in SEQ ID NO:5 of 80% or more and coding a protein having been considered to have PGA synthesis activity together with PgsB and PgsC, is being introduced into the host microorganism, wherein the host microorganism is a *Bacillus* microbe selected from *Bacillus subtilis, Bacillus licheniformis* and *Bacillus megaterium,* wherein the *pgsB* gene and the *pgsC* gene are incorporated in a plasmid self-replicable in a cell of the host microorganism and wherein multiple copies of said plasmid are contained in the cell.
2. The recombinant microorganism according to item 1, wherein the *pgsB* gene is a gene coding the following protein (a) or (b):
   (a) a protein having the amino acid sequence set forth in SEQ ID NO: 2;
   (b) a protein having an amino acid sequence, in which 2 to 80 amino acids are being deleted, substituted, added or inserted in the amino acid sequence set forth in SEQ ID NO: 2, and having amido-ligase activity.
3. The recombinant microorganism according to Item 1, wherein the *pgsB* gene is a gene having a nucteotide sequence with a genetic identity to the nucleotide sequence set forth in SEQ ID NO:1 of 80% or more and coding a protein having amido-ligase activity.
4. The recombinant microorganism according to Item 1 to 3, wherein the *pgsC* gene is a gene coding the following protein (c) or (d):
   (c) a protein having the amino acid sequence set forth in SEQ ID NO: 4; and
   (d) a protein having an amino acid sequence, in which 2 to 30 amino acids are being deleted, substituted, added or inserted in the amino acid sequence set forth in SEQ ID NO: 4, and having a function of producing poly-γ-glutamic acid in the presence of the PgsB protein coded by the *pgsB* gene.
5. The recombinant microorganism according to Item 1 to 3, wherein the *pgsC* gene is a gene having a nucleotide sequence with a genetic identity to the nucleotide sequence set forth in SEQ ID NO:3 of 80% or more and coding a protein having ability of producing PGA in the presence of the PgsB protein.
6. The recombinant microorganism according to any one of Items 1 to 5, wherein the *pgsB* gene, and the *pgsC* gene, have a transcription initiation regulatory region and/or a translation initiation regulatory region each functioning in the microorganism at a particular site upstream of the genes.
7. The recombinant microorganism according to any one of Items 1 to 6, wherein the *Bacillus* microbe is *Bacillus subtilis.*
8. The recombinant microorganism according to Item 7, wherein the *Bacillus subtilis* is *Bacillus subtilis* Marburg No. 168 strain.
9. A method of producing a poly-γ-glutamic acid, comprising the steps of:
   culturing the recombinant microorganism according to any one of Items 1 to 8, and collecting poly-γ-glutamic acid produced.
10. A method of improving a productivity in poly-γ-glutamic acid production or adjusting molecular weight of poly-γ-glutamic acid, which comprises employing a recombinant microorganism, which is obtained by introducing a *Bacillus subtilis pgsB* gene and a *Bacillus subtilis pgsC* gene into a host microorganism, wherein the host microorganism is a *Bacillus* microbe selected from *Bacillus subtilis, Bacillus licheniformis* and *Bacillus megaterium.*
11. A method of adjusting optical purity of poly-γ-glutamic acid, wherein L-isomer ratio of the poly-γ-glutamic acid produced is controlled by employing a recombinant microorganism, which is obtained by introducing a *Bacillus subtilis pgsB* gene, and a *Bacillus subtilis pgsC* gene into a host microorganism, wherein the host microorganism is a *Bacillus* microbe selected from *Bacillus subtilis, Bacillus licheniformis* and *Bacillus megaterium.*
12. The method of adjusting optical purity of poty-γ-glutamic acid according to Item 11 or the method of improving a productivity in poly-γ-glutamic acid production or adjusting Molecular weight of poly-γ-glutamic acid according to item 10, wherein the recombinant microorganism is the recombinant microorganism according to any one of Items 1 to 8.
13. A method of producing a high-molecular-weight poly-γ-glutamic acid, which comprises employing the method of adjusting molecular weight of poly-γ-glutamic acid according to Item 10 or 12.
14. A method of producing poly-γ-glutamic acid having high L-isomer ratio, which comprises employing the method of adjusting optical purity of poly-γ-glutamic acid according to Item 11 or 12.
15. Use of the recombinant microorganism according to any one of Items 1 to 8.

After intensive studies, the inventors found that it is possible to produce PGA in high productivity, to adjust molecular weight of PGA produced, and/or to adjust optical purity of PGA produced, by employing a recombinant microorganism obtained by introducing a *pgsB* gene or a gene functionally equivalent thereto and a *pgsC* gene or a gene functionally equivalent thereto into a host microorganism. The present invention was made, based on the finding above.

The recombinant microorganism according to the present disclosure is a microorganism obtained by introducing a *Bacillus subtilis pgsB* gene or a gene functionally equivalent thereto and a *Bacillus subtilis pgsC* gene or a gene functionally equivalent thereto, among a group of genes relating to synthesis of PGA *(pgsBCA* genes), into a host microorganism. However, a *Bacillus subtilis pgsA* gene or a gene functionally equivalent thereto is not being introduced into the host microorganism. Thus, the present invention is made, based on the finding that the PGA productivity is rather better when the *Bacillus subtilis pgsA* gene or a gene functionally equivalent thereto is not being introduced, in contrast with traditional understanding.

The *pgsB* gene is preferably a gene coding the following protein (a) or (b).
(a) Protein having the amino acid sequence set forth in SEQ ID NO: 2
(b) Protein having an amino acid sequence, in which one or more amino acids are being deleted, substituted, added or inserted in the amino acid sequence set forth in SEQ ID NO: 2, and having amido-ligase activity

The *pgsC* gene is preferably a gene coding the following protein (c) or (d).
(c) Protein having the amino acid sequence set forth in SEQ ID NO: 4
(d) Protein having an amino acid sequence, in which one or more amino acids are being deleted, substituted, added or inserted in the amino acid sequence set forth in SEQ ID NO: 4, and having a function of producing PGA in the present of the PgsB protein coded by the *pgsB* gene.

In addition, in the recombinant microorganism according to the present disclosure, it is preferably that the *pgsB* gene or the gene functionally equivalent thereto and the *pgsC* gene or the gene functionally equivalent thereto are bonded to the downstream of a transcription initiation regulatory region and/or a translation initiation regulatory region functioning in the host cell, the expression of which is positively regulated.

The host microorganism for the recombinant microorganism according to the present disclosure is preferably a *Bacillus* microbe. An examples of the *Bacillus* microbe includes *Bacillus subtilis.*

The method of producing PGA according to the present disclosure is a method of culturing the recombinant microorganism according to the present invention described above and collecting the PGA produced.

The method of improving the productivity of PGA according to the present disclosure is a method characterized by improving PGA productivity by employing a recombinant microorganism obtained by introducing a *Bacillus subtilis pgsB* gene or a gene functionally equivalent thereto and a *Bacillus subtilis pgsC* gene or a gene functionally equivalent thereto, among a group of genes relating to synthesis of PGA, into a host microorganism.

The method of adjusting molecular weight of PGA according to the present disclosure is a method characterized by adjusting the molecular weight of PGA (preferably making the PGA have high molecular) by employing a recombinant microorganism obtained by introducing a *Bacillus subtilis pgsB* gene or a gene functionally equivalent thereto and a *Bacillus subtilis pgsC* gene or a gene functionally equivalent thereto, among a group of the genes relating to synthesis of PGA, into a host microorganism.

The method of adjusting optical purity of PGA according to the present disclosure is a method characterized by adjusting the optical purity of PGA (preferably increasing L-isomer ratio of PGA) by employing a recombinant microorganism obtained by introducing a *Bacillus subtilis pgsB* gene or a gene functionally equivalent thereto and a *Bacillus subtilis pgsC* gene or a gene functionally equivalent thereto, among a group of genes relating to synthesis of PGA, into a host microorganism.

### BRIEF DESCRIPTION OF DRAWINGS

[Fig. 1] Fig. 1 is a flow chart for construction of a recombinant vector for producing PGA, for showing an example of the procedure of the steps of cloning the genes involved in PGA synthesis.
[Fig. 2] Fig. 2 is a flow chart showing the procedure for deleting a target gene through double crossing method by using the SOE-PCR fragments obtained in Preparation Example 5.
[Fig. 3] Fig. 3 is a flow chart for construction of a recombinant vector for producing PGA, for showing the procedure of the steps of cloning the genes involved in PGA synthesis in Preparation Examples 2 and 3.
[Fig. 4] Fig. 4 is a flow chart for construction of a recombinant vector for producing PGA, for showing the procedure of the steps of cloning the genes involved in PGA synthesis in Preparation Example 4.

Other and further features and advantages of the invention will appear more fully from the following description, appropriately referring to the accompanying drawings.

### BEST MODE FOR CARRYING OUT THE INVENTION

Hereinafter, the present disclosure will be described in detail.

The recombinant microorganism according to the present disclosure is a microorganism obtained by introducing a *Bacillus subtilis pgsB* gene or a gene functionally equivalent thereto and a *Bacillus subtilis pgsC* gene or a gene functionally equivalent thereto, among a group of genes relating to synthesis of PGA, into a host microorganism, but not introducing a *Bacillus subtilis pgsA* gene into the host microorganism. The recombinant microorganism has ability of producing poly-γ-glutamic acid.

Herein, the group of genes relating to synthesis of PGA include the *pgsB* gene (BG12531: also called *ywsC* gene or *capB* gene), the *pgsC* gene (BG12532: also called *ywtA* gene or *capC* gene) and the *pgsA* gene (BG12533: also called *ywtB* gene or *capA* gene) in *Bacillus subtilis. Bacillus subtilis* Marburg No.168 strain, which is widely used as a host microorganism for gene recombinant, has these *pgsB* gene, *pgsC* gene and *pgsA* gene on its chromosome, but does not have ability of producing PGA. Similarly, *Bacillus subtilis* var. *natto* has these *pgsB* gene, *pgsC* gene and *pgsA* gene on its chromosome (genome) and has ability of producing PGA. The name and the number of each gene are described, according to the *Bacillus subtilis* genome data disclosed on Internet (http://bacillus.genome.ad.jp/, updated on Jan. 18) by JAFAN [Japan Functional Analysis Network for Bacillus subtilis (BSORF DB)].

The nucleotide sequence of the *pgsB* gene is set forth in SEQ ID NO: 1, and the amino acid sequence of the PgsB protein coded by the *pgsB* gene is set forth in SEQ ID NO: 2. In the present invention, the *pgsB* gene is not limited to the gene having the nucleotide sequence set forth in SEQ ID NO: 1, and examples thereof include a gene coding a protein having a modified amino acid sequence of SEQ ID NO: 2 in which one or more amino acids are being deleted, substituted, added or inserted and having amido-ligase activity by using glutamic acid as substrate. The number of amino acids modified then may be, for example, 2 to 80, preferably 2 to 40, more preferably 2 to 20, still more preferably 2 to 10, and most preferably 2 to 5.

In the present invention, the *pgsB* gene includes a gene having a nucleotide sequence with a genetic identity to the nucleotide sequence set forth in SEQ ID NO: 1 of 80% or more, preferably 90% or more, more preferably 95% or more, more preferably 96% or more, still more preferably 97% or more, most preferably 98% or more, and particularly preferably 99% or more; and coding a protein having amido-ligase activity. The homology of the nucleotide sequence is calculated through the Lipman-Pearson method (see Lipman, DJ., Pearson, WR.: Science, 227, pp. 1435-1441 (1985)). Specifically, the homology can be determined through use of a homology analysis (Search homology) program of genetic information processing software Genetyx-Win (Software Development Co., Ltd.) (Unit size to compare (ktup)=2). When a reaction solution containing glutamic acid and ATP as substrates and additionally manganese chloride is used, the amido-ligase activity can be determined by measuring the PGA generated in the reaction solution [see, for example, Urushibata, Y., et al.: J. Bacteriol., 184, pp. 337-343 (2002)].

In the present invention, the *pgsB* gene includes a gene capable of hybridizing with a complementary sequence to the nucleotide sequence set forth in SEQ ID NO: 1 under a stringent condition, and coding a protein having amido-ligase activity. The "stringent condition" above is, for example, the condition described in "Molecular Cloning - A LABORATORY MANUAL THIRD EDITION" [Joseph Sambrook, David W. Russell, Cold Spring Harbor Laboratory Press (2001)]. It is, for example, a hybridization condition of a gene with a probe by incubation thereof in a solution containing 6 x SSC (1 x SSC composition: 0.15 M sodium chloride and 0.015 M sodium citrate, pH 7.0), 0.5% SDS, 5 x Denhardt and 100 mg/mL herring sperm DNA at 65°C for 8 to 16 hours.

The amido-ligase activity described above is an activity to produce PGA extracellularly when the *pgsB* gene is introduced together with the *pgsC* gene into a host microorganism, as will be described below in Examples in detail. The ability of producing PGA of a particular microorganism can be determined, for example, by a method of visually observing the semitransparent viscous substance formed around a colony when the microorganism is cultured on a PGA production agar medium containing glutamic acid or the salt thereof, a method of detecting PGA by SDS-PAGE [see, for example, Yamaguchi, F., et al.: Biosci. Biotechnol. Biochem., 60, pp. 255-258 (1996)], a method of quantitatively determining PGA after acid hydrolysis by using an amino acid analyzer [see, for example, Ogawa, Y., et al.: Biosci. Biotechnol. Biochem., 61, pp. 1684-1687 (1997)], a quantitative method of determining the dry weight after purification of the culture solution [see, for example, Ashiuchi, M., et al.: Biochem. Biophys. Res. Commun., 263: pp. 6-12 (1999)], or a quantitative/qualitative method by HPLC (high-performance liquid chromatography) by using a gel filtration column [see, for example, Kubota, H., et al.: Biosci. Biotechnol. Biochem., 57, pp. 1212-1213 (1993)].

The nucleotide sequence of the *pgsC* gene is set forth in SEQ ID NO: 3, and the amino acid sequence of the PgsC protein coded by the *pgsC* gene is set forth in SEQ ID NO: 4. In the present invention, the *pgsC* gene is not limited to the gene having the nucleotide sequence set forth in SEQ ID NO: 3, and examples thereof includes a gene coding a protein having a modified amino acid sequence of SEQ ID NO: 4 in which one or more amino acids are being deleted, substituted, added or inserted and having ability of producing PGA in the presence of the PgsB protein described above. The number of amino acids modified then may be, for example, 2 to 30, preferably 2 to 15, more preferably 2 to 8, still more preferably 2 to 2, and most preferably 2.

In the present invention, the *pgsC* gene includes a gene having a nucleotide sequence having a genetic identity to the nucleotide sequence set forth in SEQ ID NO: 3 of 80% or more, preferably 90% or more, more preferably 95% or more, more preferably 96% or more, still more preferably 97% or more, most preferably 98% or more and particularly preferably 99% or more; and coding a protein having ability of producing PGA in the presence of the PgsB protein described above. The identity of nucleotide sequence is the same as that defined above.

In the present invention, the *pgsC* gene includes a gene capable of hybridizing with a complementary sequence to the nucleotide sequence set forth in SEQ ID NO: 3 under a stringent condition, and coding a protein having ability of producing PGA in the presence of the PgsB protein described above. The "stringent condition" is also the same as that defined above.

The ability of the PgsC protein of producing PGA in the presence of the PgsB protein described above means to produce PGA extracellularly when the *pgsC* gene is expressed with the *pgsB* gene in a host microorganism. As described in detail below in Examples and Reference Example, the present invention is based on the finding by the present inventors that a host microorganism expressing the *pgsB* gene alone does not produce PGA, while that expressing both the *pgsB* gene and *pgsC* gene do produce PGA.

The recombinant microorganism according to the present invention is not limited to the above-described host microorganism containing the introduced *pgsB* and *pgsC* genes derived from *Bacillus subtilis,* and may include, for example, host microorganisms having an introduced gene functionally equivalent to the *pgsB* gene (*pgsB* homologous gene) and an introduced gene functionally equivalent to the *pgsC* gene (*pgsC* homologous gene), among a group of genes relating to synthesis of PGA derived from microorganisms other than *Bacillus subtilis.* The *pgsB* homologous gene and the *pgsC* homologous gene can be isolated and identified from a known microorganism capable of producing PGA by a common method. Examples of the microorganism having the ability of producing PGA include, in addition to *Bacillus subtilis, Bacillus licheniformis, Bacillus megaterium, Bacillus anthracis, Bacillus halodurans, Natrialba aegyptiaca,* and *Hydra.* The *pgsB* homologous gene and the *pgsC* homologous gene can be isolated and identified from these microorganisms. The *pgsB* homologous gene can be isolated, for example, by extracting genomic DNA from any of the microorganisms and performing Southern hybridization while the polynucleotide having the nucleotide sequence set forth in SEQ ID NO: 1 above is used as probe. The *pgsC* homologous gene can also be isolated similarly. Further, in recent rapid progress in genome sequencing, the *Bacillus subtilis pgsB* homologous gene as defined above can be isolated and identified even in a microorganism incapable of producing PGA, based on the genome sequence information. The *Bacillus subtilis pgsC* homologous gene can be isolated in the same manner as described above.

The recombinant microorganism according to the present invention is prepared by introducing the *pgsB* gene or the *pgs*B homologous gene described above and the *pgsC* gene or the *pgsC* homologous gene into a host microorganism.

The host microorganism for use in the present disclosure is not particularly limited, and typical examples thereof include *Bacillus* microbes such as *Bacillus licheniformis, Bacillus megaterium, Bacillus anthracis, Bacillus halodurans,* and *Bacillus subtilis; Clostridium* microbes, *Corynebacterium* microbes, *Escherichia coli,* and yeasts, and *Bacillus* microbes are preferable. In particular, *Bacillus subtilis* is preferable, because the entire genome information is known, the genetic and genomic engineering technologies thereof are established, and there are available many microbial strains that are established as safe and favorable host microorganisms.

The host cell to be chosen may be a wild-type cell or a mutant-type strain containing a particular mutation introduced into the wild-type cell. In particular, among the mutant strains, it would be possible to obtain favorable effect, by employing a mutant strain, in which a gene coding a enzyme for decomposing PGA (a gene of enzyme for decomposing PGA) is being deleted, as the host microorganism. A known gene of enzyme for decomposing PGA in *Bacillus subtilis* is a *ggt* gene [see, for example, Kimura, K., et al.: Microbiology, 150, pp. 4115-4123 (2003)]. The nucleotide sequence of the *ggt* gene is set forth in SEQ ID NO: 7, and the amino acid sequence of the enzyme for decomposing PGA coded by the *ggt* gene is set forth in SEQ ID NO: 8. When a microorganism other than *Bacillus subtilis* is used as the host, use of a mutant strain with the *ggt-*equivalent gene deleted is preferable. In the present invention, the *ggt* gene is not limited to the gene having the nucleotide sequence set forth in SEQ ID NO: 7, and examples thereof include a gene coding a protein having a modified amino acid sequence of SEQ ID NO: 8 in which one or more amino acids are being deleted, substituted, added or inserted and having activity of decomposing PGA. The number of amino acids modified then may be, for example, 2 to 120, preferably 2 to 60, more preferably 2 to 30, still more preferably 2 to 15, and most preferably 2 to 8. In the present invention, the *ggt* gene includes a gene having a nucleotide sequence having a genetic identity to the nucleotide sequence set forth in SEQ ID NO: 7 of 80% or more, preferably 90% or more, more preferably 95% or more, more preferably 96% or more, still more preferably 97% or more, most preferably 98% or more and particularly preferably 99% or more; and coding a protein having activity of decomposing PGA. The identity of nucleotide sequence is the same as that defined above.

In the present disclosure, the *ggt* gene includes a gene capable of hybridizing with a complementary sequence to the nucleotide sequence set forth in SEQ ID NO: 8 under a stringent condition, and coding a protein having activity of decomposing PGA. The "stringent condition" is also the same as that defined above. The *ggt* gene or the homologous gene thereof can be deleted properly by using a known method. For example, it is possible to introducing a cyclic recombinant plasmid, which is obtained by cloning a DNA fragment containing a part of the *ggt* gene into a suitable plasmid (vector), into a host microorganism cell and inactivates the target gene on the host microorganism genome by fragmenting it by homologous mutation in a partial region of the target gene [see Leenhouts, K., et al.: Mol. Gen. Genet., 253, pp. 217-224 (1996)].

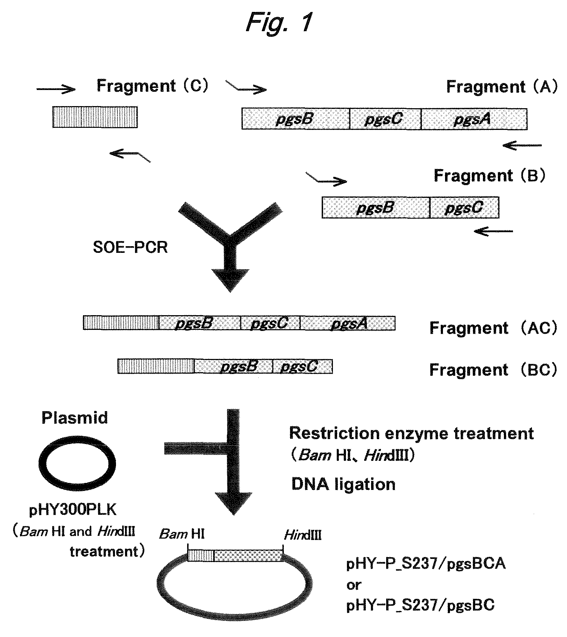

In particular, when *Bacillus subtilis* is used as the host microorganism for construction of the microorganism according to the present invention, there are some reported methods of deleting or inactivating the target gene by homologous recombination [see, for example, Itaya, M., Tanaka, T.: Mol. Gen. Genet., 223, pp. 268-272 (1990)], and it is possible to obtain the desired host microorganism by repeating these methods. Inactivation of random genes can also be performed by use of a mutagenetic agent or by irradiation on host microorganism with ultraviolet ray, γ-ray or the like. In such a case, it is also possible to obtain similar effect, for example, by mutagenesis by means of base substitution of nucleotides or insertion of nucleotides, or deletion of part of the gene, by similar mutagenesis in the regulatory regions such as promoter region, or by similar mutagenesis of the genes dependent on the expression control of the *ggt* gene. It is also possible to delete the *ggt* gene on genome by a more efficient method of constructing a straight-chain DNA fragment containing the upstream and downstream regions of the *ggt* gene but not containing the *ggt* gene by SOE (splicing by overlap extension)-PCR method [see, for example, Horton, RM., et al.: Gene, 77, pp. 61-68 (1989)], introducing it into a host microorganism cell, and thus, causing homologous recombination by double crossing at two sites outside the *ggt gene* on the host microorganism genome (see Fig. 1).

The host cell chosen may be a microorganism inherently having ability of producing PGA or a microorganism inherently having no ability of producing PGA. It may be thus a microorganism having the *pgsBCA* gene on chromosome (genome) and having ability of producing PGA, a microorganism having the *pgsBCA* gene on chromosome but no ability of producing PGA, or a microorganism having no *pgsBCA* gene at all. Introduction of the *pgsB* gene or the *pgsB* homologous gene and the *pgsC* gene or the *pgsC* homologous gene described above into a microorganism having ability of producing PGA leads to increase in ability of producing PGA and/or in the molecular weight of PGA produced. On the other hand, introduction of the *pgsB* gene or the pgsB homologous gene and the *pgsC* gene or the *pgsC* homologous gene described above into a microorganism having no ability of producing PGA leads to addition of the ability of producing PGA.

It is possible to use, as the host cell, a microorganism having ability of producing PGA which is obtained by introducing therein a transcription initiation regulatory region and/or a translation initiation regulatory region functioning in the microorganism, at the sites upstream of the *pgsBCA* gene or a gene functionally equivalent thereto of *Bacillus subtilis* on chromosome (genome).

The transcription initiation regulatory region and/or the translation initiation regulatory region functioning in the host microorganism according to the present invention (hereinafter, referred to as "regulatory region") are preferably bound thereto in a suitable form, and the transcription initiation regulatory region and the translation initiation regulatory region are preferably bound to each other.

The regulatory region is more preferably a region that can constitutively express downstream genes bound to the region and increase the expression amount of the downstream genes in host cell. Examples of the regulatory region include a regulatory region of an α-amylase gene, a regulatory region of a protease gene, a regulatory region of an *aprE* gene, a regulatory region of a *spoVG* gene and a regulatory region of a *rapA* gene derived from *Bacillus* microbes; a regulatory region of a cellulase gene derived from *Bacillus* sp. KSM-S237 strain; and a regulatory region of a kanamycin resistance gene and a regulatory region of a chloramphenicol resistance gene derived from *Staphylococcus aureus.*

In introduction of the regulatory region on the genome upstream of the *pgsBCA* gene or the gene functionally equivalent thereto, part or all of the inherent regulatory region for the *pgsBCA* gene or the gene functionally equivalent thereto of *Bacillus subtilis* is used for substitution.

The substitution to the regulatory region may be performed by any known method, for example, by homologous recombination (e.g., the method described in Mol. Gen. Genet., 223, 268, 1990).

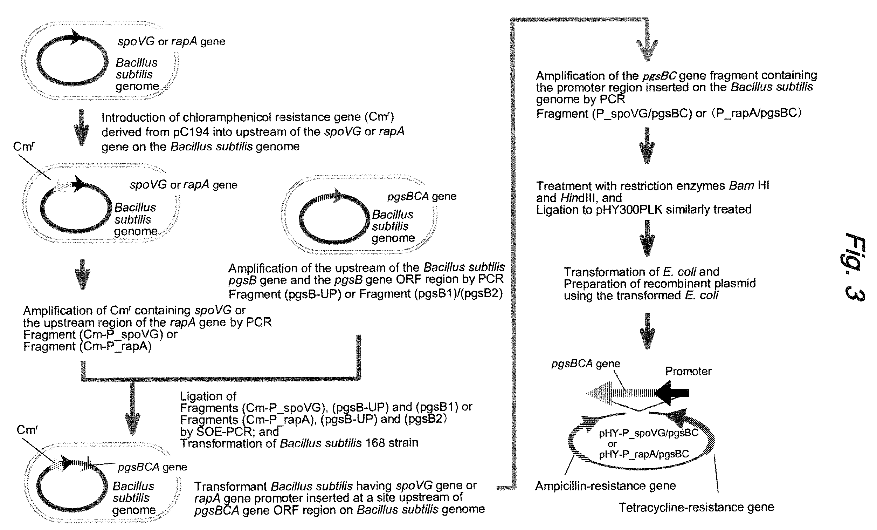

For example, first, a DNA fragment containing the upstream region of the transcription initiation regulatory region inherent to the *pgsBCA* gene and a drug-resistance gene fragment are bound to the sites upstream of the DNA fragment containing the regulatory region, and a DNA fragment containing part or all of the translation initiation regulatory region of the *pgsBCA* gene or part or all of the structural gene region of the *pgsBCA* gene is bound to the site downstream the DNA fragment containing the regulatory region, by a common method such as SOE-PCR method. In this way, obtained is a DNA fragment containing a DNA fragment having the upstream region of the inherent transcription initiation regulatory region of the *pgsBCA* gene, a drug-resistance gene fragment, a DNA fragment having the regulatory region, and part or all of the translation initiation regulatory region of the *pgsBCA* gene and the structural gene region, and part or all of the structural gene region of the *pgsBCA* gene, in that order (see Fig. 3).

Subsequent incorporation of the DNA fragment into host microorganism cell by a common method results in homologous double-crossing recombination at two sites with the upstream region of the inherent transcription initiation regulatory region of the *pgsBCA* gene on the host microorganism genome, and part or all of the translation initiation regulatory region of the *pgsBCA* gene and the structural gene region or part or all of the structural gene region of the *pgsBCA* gene. As a result, the transformant having its inherent regulatory regions substituted with the regulatory regions above can be separated by employing the drug-resistance gene as indicator. The regulatory region introduced to the genome upstream of the pgsBCA gene is thus retained as it is genetically stabilized.

The recombinant microorganism according to the present invention does not contain the *pgsA* gene introduced, among a group of the genes relating to synthesis of PGA in the host microorganism. The nucleotide sequence of the *pgsA* gene is set forth in SEQ ID NO: 5, while the amino acid sequence of the protein coded by the *pgsA* gene is set forth in SEQ ID NO: 6. The protein coded by the *pgsA* gene is suggested to be a protein involved in extracellular release of PGA (see, for example, the Non-Patent Document 5). However, the function thereof remains unresolved even now. Characteristically, the recombinant microorganism according to the present invention is superior in PGA productivity, even though it contains no *pgsA* gene introduced. The *pgsA* gene in the present invention is not limited to the gene having the nucleotide sequence set forth in SEQ ID NO: 5, and examples thereof includes a gene coding a protein having a modified amino acid sequence of SEQ ID NO: 6 in which one or more amino acids are being deleted, substituted, added or inserted, in which the protein has been considered to have PGA synthesis activity together with the PgsB and the PgsC. The number of amino acids modified then may be, for example, 2 to 80, preferably 2 to 40, more preferably 2 to 20, still more preferably 2 to 10, and most preferably 2 to 5. In the present invention, the *pgsA* gene includes a gene having a nucleotide sequence having an identity to the nucleotide sequence set forth in SEQ ID NO: 5 of 80% or more, preferably 90% or more, more preferably 95% or more, more preferably 96% or more, still more preferably 97% or more, most preferably 98% or more and particularly preferably 99% or more, and coding a protein having been considered to have PGA synthesis activity together with the PgsB and the PgsC. The identity of nucleotide sequence is the same as that defined above.

In the present invention, the *pgsA* gene includes a gene capable of hybridizing with a complementary sequence to the nucleotide sequence set forth in SEQ ID NO: 5 under a stringent condition, and coding a protein having been considered to have PGA synthesis activity together with the PgsB and the PgsC. The "stringent condition" is also the same as that defined above.

The phrase "no *pgsA* gene is being introduced into the host microorganism" refers to that no gene coding the PgsA protein functioning in the PGA synthesis process is introduced. Accordingly in the case where a gene having a higher identity with the gene set forth in SEQ ID NO: 5 is introduced, when the gene is not expressed or the protein produced by expression of the gene does not function in the PGA synthesis process, such a microorganism is also included in the technical scope of the present invention.

Preferably, in the recombinant microorganism according to the present invention, the *pgsB* gene or the *pgsB* homologous gene and the *pgsC* gene or the *pgsC* homologous gene described above have a transcription initiation regulatory region and a translation initiation regulatory region functioning in the host cell and positively expression-controlled that are bound to the sites upstream of the genes. The regulatory regions are more preferably regions expressing the downstream bound genes and increasing the expression amounts of the genes in host cell, and those expressing the downstream gene constitutively or/and in a greater amount are particularly preferable. Examples of the regulatory regions include, but are not limited to, a regulatory region of an α-amylase gene, a regulatory region of a protease gene, a regulatory region of an *aprE* gene, a regulatory region of a *spoVG* gene and a regulatory region of a *rapA* gene derived from *Bacillus* microbes; a regulatory region of a cellulase gene of *Bacillus* sp. KSM-S237 strain; and a regulatory region of a kanamycin resistance gene and a regulatory region of a chloramphenicol resistance gene derived from *Staphylococcus aureus.*

The nucleotide sequence of the regulatory region of the cellulase gene of the *Bacillus* sp. KSM-S237 strain, i.e., the regulatory region upstream by 0.4 to 1.0 kb of the translation initiation point of the cellulase gene, described above, is set forth in SEQ ID NO: 9. The regulatory region is not limited to that having the nucleotide sequence set forth in SEQ ID NO: 9, and it includes, for example, a nucleotide sequence having a genetic identity to the nucleotide sequence set forth in SEQ ID NO: 9 of 70% or more, preferably 80% or more, more preferably 90% or more, more preferably 96% or more, still more preferably 97% or more, most preferably 98% or more and particularly preferably 99% or more. The identity of nucleotide sequence is the same as that defined above.

The nucleotide sequence of the regulatory region of the *spoVG* gene (BG10112) derived from *Bacillus subtilis* is set forth in SEQ ID NO: 10. The regulatory region is not limited to that having the nucleotide sequence set forth in SEQ ID NO: 10, and it includes, for example, a nucleotide sequence having a genetic identity to the nucleotide sequence set forth in SEQ ID NO: 10 of 70% or more, preferably 80% or more, more preferably 90% or more, more preferably 96% or more, still more preferably 97% or more, most preferably 98% or more and particularly preferably 99% or more. The identity of nucleotide sequence is the same as that defined above.

The nucleotide sequence of the regulatory region of the *rapA* gene (BG10652) derived from *Bacillus subtilis* is set forth in SEQ ID NO: 11. The regulatory region is not limited to that having the nucleotide sequence set forth in SEQ ID NO: 11, and it includes, for example, a nucleotide sequence having a genetic identity to the nucleotide sequence set forth in SEQ ID NO: 11 of 70% or more, preferably 80% or more, more preferably 90% or more, more preferably 96% or more, still more preferably 97% or more, most preferably 98% or more and particularly preferably 99% or more. The identity of nucleotide sequence is the same as that defined above.

A common plasmid (vector) may be used in introducing the *pgsB* gene or the *pgsB* homologous gene and the *pgsC* gene or the *pgsC* homologous gene described above into a host microorganism. The plasmid may be selected properly according to the kind of the host microorganism to which the genes are to be introduced. Examples thereof, when *Bacillus subtilis* is the host, include pT181, pC194, pUB110, pE194, pSN2, and pHY300PLK. The plasmid to be selected is preferably a plasmid self-replicable in the host cell, and more preferably, there are multiple copies of the plasmid contained in cell. The copy number of plasmids with respect to the host genome (chromosome) is favorably 2 or more and 100 or less, preferably 2 or more and 50 or less, and more preferably 2 or more and 30 or less. A common method such as protoplast method [see, for example, Chamg, S., Cohen, SH.: Mol. Gen. Genet., 168, pp. 111-115 (1979)] and competent cell method [see, for example, Young, FE., Spizizen, J.: J. Bacteriol., 86, pp. 392-400 (1963)] may be used in transformation with the expression vector.

It is possible to produce PGA at excellent productivity by employing the recombinant microorganism according to the present invention described above. The PGA produced can be used in various applications such as cosmetics, medicines, foods, water purifiers, moisturizing materials, thickeners, and others. In particular, the recombinant microorganism according to the present invention, which improves PGA productivity significantly, compared to that by conventional genetic engineering technologies, allows drastic reduction of the production cost for PGA that is used in the applications above.

In particular, in production of PGA by employing the recombinant microorganism according to the present invention, the recombinant microorganism is first cultured in a suitable medium and the PGA extracellularly produced is collected. The medium for use is, for example, a medium in the composition containing carbon sources such as glycerol, glucose, fructose, maltose, xylose, arabinose, and various organic acids, nitrogen sources such as various amino acids, polypeptone, tryptone, ammonium sulfate, and urea, inorganic salts such as sodium salt and other needed nutrient sources, trace amounts of metal salts, and the like. The medium may be a synthetic medium or a natural medium.

In particular, for improvement in PGA productivity, it is preferable to use a medium containing glutamic acid in an amount of more than that needed for growth of the recombinant microorganism to be cultured. Specifically, it is preferable to add it, as sodium glutamate, to the medium, and the concentration therein is, for example, 0.005 to 600 g/L, preferably 0.05 to 500 g/L, more preferably 0.1 to 400 g/L, and most preferably 0.5 to 300 g/L. An excessively low sodium glutamate concentration in the medium may lead to complete consumption of the glutamic acid in the medium by the recombinant microorganism and consequently to insufficient improvement in PGA productivity. Alternatively, an excessively high glutamic acid concentration in the medium may lead to a problem of precipitation of sodium glutamate and other medium components if it is higher than the saturation solubility of sodium glutamate.

For collection of the PGA accumulated in the medium, it is necessary to remove the microorganism microbe that has produced PGA, for example, by means of centrifugation, ultrafiltration membrane, electrodialysis, isoelectric focusing of pH to the isoelectric point of PGA, or the like, and these methods may be used in combination.

In this way, it is possible to produce PGA by employing the recombinant microorganism according to the present invention. As will be described in Examples below, the recombinant microorganism according to the present invention shows a high productivity of 1.5 g/L or more, and even the recombinant microorganism having the introduced *Bacillus subtilis pgsB* gene or gene functionally equivalent thereto and *Bacillus subtilis pgsC* gene or gene functionally equivalent thereto but having no *Bacillus subtilis pgsA* gene or gene functionally equivalent thereto shows favorable PGA productivity, compared to microorganisms capable of producing PGA obtained by conventional genetic engineering technology. Thus, the recombinant microorganism according to the present invention is useful in industrial production of PGA.

As will be described in Example below, introduction of the *Bacillus subtilis pgsB* gene or gene functionally equivalent thereto and the *Bacillus subtilis pgsC* gene or gene functionally equivalent thereto into a microorganism having ability of producing PGA results in making the PGA produced have high molecular weight. Thus, introduction of these genes into the microorganism with ability of producing PGA enables regulation of the molecular weight of PGA produced, and moreover, use of the recombinant microorganism obtained enables efficient production of PGA having a desired molecular weight.

In particular, when a microorganism having the regulatory region of the cellulase gene derived from *Bacillus* sp. KSM-S237 strain bound to the upstream regulatory region of the *pgsBCA* gene is used, it is possible to produce a high-molecular-weight PGA having a weight-averaged molecular weight of approximately 7,000,000 to 7,800,000. Introduction of the *Bacillus subtilis pgsB* gene or gene functionally equivalent thereto and the *Bacillus subtilis pgsC* gene or gene functionally equivalent thereto to this microorganism allows control of the molecular weight of PGA, i.e., increase in PGA molecular weight preferably to 110 to 115%.

When a microorganism having the regulatory region of the *rapA* gene bound to the upstream regulatory region of the *pgsBCA* gene is used as the host cell, it is possible to produce PGA having medium-molecular weight, e.g., a weight-averaged molecular weight of approximately 590,000 to 600,000. When a microorganism having the introduced *Bacillus subtilis pgsB* gene or gene functionally equivalent thereto and the introduced *Bacillus subtilis pgsC* gene or gene functionally equivalent thereto is used, it is possible to adjust the molecular weight of PGA, i.e., increase in the molecular weight of PGA produced preferably to 1,200 to 1,600%.

Further, introduction of the *Bacillus subtilis pgsB* gene or gene functionally equivalent thereto and the *Bacillus subtilis pgsC* gene or gene functionally equivalent thereto into a microorganism having no ability of producing PGA enables control of the L-glutamic acid content to preferably 80% or more, more preferably 90% or more. In addition, according to the present invention, it is possible to produce PGA high in optical purity (e.g., at an optical purity of preferably 80 to 100%, more preferably 90 to 100%).

By employing the recombinant microorganism according to the present invention, it is possible to produce PGA having a molecular weight varying properly according to applications, and to reduce the production cost for the PGA used in various applications drastically, and to produce PGA high in optical purity. Thus, the recombinant microorganism is useful in industrial production of PGA.

### EXAMPLES

Hereinafter, the present invention will be described more in detail with reference to Examples, but it should be understood that the technological scope of the present invention is not particularly limited by the following Examples. The name, the sequence and the SEQ ID NO: of each of the primers used in Examples are shown in Table 1. The underlined region in the nucleotide sequence shown in Table 1 indicates the sequence of the restriction site added.

**Table 1**

| Primer | Nucleotide sequence | SEQ ID NO: |
|---|---|---|
| pgsB-F | | 12 |
| pgsA-R | CGAAGCTTAGATGGCTTTGACAAATTTCATC | 13 |
| pgsC-R | CCCAAGCTTGACCTTCGGCGTTTCCGCT | 14 |
| pgsB-R | CCCAAGCTTGGCAGCGAATTTTCTGCGTCC | 15 |
| P_S237-FW | | 16 |
| P S237-RV | CATCATATTACCTCCTAAATATTTTTAAAGTA | 17 |
| ggt-F | TCCTTCATGTCTTTCGTATA | 18 |
| ggt/Cm-R | | 19 |
| ggt/Cm-F | | 20 |
| ggt-R | TAGCCAATATCACTTTTCATC | 21 |
| CmFW | CAACTAAAGCACCCATTAGTTCAACAAACG | 22 |
| CmRV | CTTCAACTAACGGGGCAGGTTAGTGAC | 23 |
| P-spoVG FW2 | ATGAAGTTTCGTCGCAGCGG | 24 |
| P-spoVG/Cm R | | 25 |
| P_spoVG/Cm F | | 26 |
| P spoVG RV | CATAGTAGTTCACCACCTTTTCCCTATA | 27 |
| comp_Cm FW | | 28 |
| comp_Cm RV | | 29 |
| comp P-spoVG R/BSpgsB atg FW | | 30 |
| pgsC FW | GTCTGCATTTCCCCCTAGCTTACG | 31 |
| pgsB/Cm F | | 32 |
| pgsB RV | AAGGGTTTGTGATATCCGG | 33 |
| BamHI_PspoVG FW | | 34 |
| rapA FW | TGAAAAGATGCGTGCATTTC | 35 |
| P_rapA/Cm R | | 36 |
| P_rapA/Cm F | | 37 |
| P rapA RV | CATTTAATCCCCCCTTTTGAATT | 38 |
| comp_P-rapA R/BSpgsB atg FW | | 39 |
| BamHI_PrapA FW | | 40 |

### [Preparation Example 1] Construction of vector (1)

A method of cloning genes relating to synthesis of PGA is shown in this Preparation Example (see Fig. 1).

A chromosome DNA, serving as a template, prepared from *Bacillus* sp. KSM-366 strain (FERM BP-6262) and a primer set of pgsB-F (SEQ ID NO: 12) and pgsA-R (SEQ ID NO: 13) shown in Table 1 were used to prepare a 2.9 kb DNA fragment (A) of *pgsBCA* gene. The nucleotide sequence of the *pgsB* gene of the *Bacillus* sp. KSM-366 strain is set forth in SEQ ID NO: 47, the amino acid sequence of the protein coded by the *pgsB* gene is set forth in SEQ ID NO: 48, the nucleotide sequence of the *pgsC* gene is set forth in SEQ ID NO: 49, the amino acid sequence of the protein coded by the *pgsC* gene is set forth in SEQ ID NO: 50, the nucleotide sequence of the *pgsA* gene is set forth in SEQ ID NO: 51, and the amino acid sequence of the protein coded by the *pgsA* gene is set forth in SEQ ID NO: 52. Separately, a primer set of pgsB-F and pgsC-R (SEQ ID NO: 14) shown in Table 1 was used to prepare a 1.7 kb DNA fragment (B) of *pgsBC* gene. Yet separately, a chromosome DNA, serving as a template, prepared from *Bacillus* sp. KSM-S237 strain (FERM BP-7875) and a primer set of P_S237-FW (SEQ ID NO: 16) and P_S237-RV (SEQ ID NO: 17) shown in Table 1 were used to prepare a 0.6 kb DNA fragment (C) of the promoter region of the cellulase gene derived from KSM-S237 strain (see JP-A-2000-210081).

Then, a 3.5 kb composite DNA fragment (AC) containing the fragments (A) and (C) was prepared by using the thus-prepared fragments (A) and (C) as templates and a primer set of P_S237-FW and pgsA-R by SOE-PCR method. Similarly, a 2.3 kb composite DNA fragment (BC) containing the fragments (B) and (C) was obtained by using the fragments (B) and (C) as templates and a primer set of P_S237-FW and pgsC-R by SOE-PCR method. These DNA fragments (AC) and (BC) were treated with restriction enzymes *Bam*HI and *Hin*dIII, respectively, and ligated with (bound to) a plasmid vector pHY300PLK (Takara) previously treated with the same restriction enzymes by using DNA Ligation kit Ver. 2 (Takara).

The thus-prepared legation sample was used to transform *Escherichia coli* (HB101 strain) by a competent cell method and cultured on, and a colony formed on a LB agar medium (LBTc agar medium) containing a 15-ppm tetracycline hydrochloride salt (SIGMA-ALDRICH) was collected as a transformant. The transformant obtained was grown once again on the LBTc agar medium, and recombinant plasmids were prepared from the obtained microbe by using High Pure Plasmid Isolation Kit (Roche Diagnostics). The recombinant plasmids were treated with the restriction enzymes *Bam*HI and *Hin*dIII, and insertion of a desirable DNA fragment (AC) or (BC) into the *Bam*HI and *Hin*dIII restriction sites of the plasmid vectors was confirmed by electrophoresis.

In this Preparation Example, recombinant plasmids confirmed to contain the DNA fragments (AC) and (BC) were designated recombinant vectors for producing PGA pHY-P_S237/pgsBCA and pHY-P_S237/pgsBC, respectively. The PCR reaction was carried out by using GeneAmp 9700 (PE Applied Biosystems) and TaKaRa LA Taq (Takara) according to the protocols attached to the kits.

### [Preparation Example 2] Construction of vector (2)

A method of cloning genes relating to synthesis of is shown in this Preparation Example (see Fig. 3).

A chromosome DNA, serving as a template, prepared from *Bacillus subtilis* Marburg No. 168 stain (*Bacillus subtilis* 168 strain) and a primer set of P_spoVG FW2 (SEQ ID NO: 24) and P_spoVG/Cm R (SEQ ID NO: 25) shown in Table 1 were used to prepare an upstream 0.9 kb DNA fragment (spoVG-UP) for insertion of the chloramphenicol-resistant gene (Cm^{r}) derived from the plasmid pC194 [see, for example, Horinouchi, S., Weisblum, B.: J. Bacteriol., 150, pp. 815-825 (1982)] to a site approximately 0.6 kb upstream of *spoVG* gene ORF. Similarly, a primer set of P_spoVG/Cm F (SEQ ID NO: 26) and P_spoVG RV (SEQ ID NO: 27) shown in Table 1 was used to prepare a downstream 0.6 kb DNA fragment (spoVG-DW) (then, the primer P_spoVG RV was designed to modify the initiation codon of the spoVG gene ORF from gtg to atg). Separately, the plasmid pC194, serving as a template, and a primer set comp_Cm FW (SEQ ID NO: 28) and comp_Cm RV (SEQ ID NO: 29) shown in Table 1 were used to prepare a chloramphenicol-resistant gene 0.9 kb DNA fragment (comp_Cm).

Subsequently, the thus-prepared fragments (spoVG-UP), (spoVG-DW) and (comp_Cm), as templates, and a primer set of P_spoVG FW2 and P_spoVG RV were used to prepare a composite 2.4 kb DNA fragment (Cm-P_spoVG) of the three fragments by SOE-PCR method. The thus-prepared DNA fragment was used to transform a *Bacillus subtilis* 168 strain by the competent cell method (described above), and a colony growing on a LB agar medium (LBCm agar medium) containing 10 ppm of chloramphenicol (SIGMA-ALDRICH) was separated as a transformant.

Then, a genome DNA extracted from the thus-prepared transformant, serving as a template, and a primer set of comp_Cm FW and P_spoVG RV shown in Table 1 were used to prepare a 1.5 kb DNA fragment (P-spoVG-Cm2) containing the chloramphenicol-resistant gene and a promoter region of the *spoVG* gene by PCR. Separately, a primer set of comp_P-spoVG R/BSpgsB atg FW (SEQ ID NO: 30) and pgsC FW (SEQ ID NO: 31) and a genome DNA sample prepared from *Bacillus subtilis* 168 strain, as a template, were used to prepare a *pgsB* gene 1.2 kb DNA fragment (pgsB1) by PCR; and a primer set of pgsB/Cm F (SEQ ID NO: 32) and pgsB RV (SEQ ID NO: 33) and the template were used to prepare an upstream 1.1 kb DNA fragment (pgsB-UP) of *pgsB* gene ORF. Further, the thus-prepared three fragments (pgsB1), (P-spoVG-Cm2) and (pgsB-UP), as templates, and a primer set of pgsC FW and pgsB RV were used to prepare a 3.6 kb DNA fragment by SOE-PCR.

Subsequently, the thus-prepared DNA fragment was used to transform *Bacillus subtilis* 168 strain by the competent cell method, and a colony growing on a LBCm agar medium was separated as a transformant (hereinafter, the thus-obtained recombinant *Bacillus subtilis* is referred to as 1cp-P_spoVG/pgsBCAcm strain). The genome DNA sample extracted from the transformant obtained, as a template, and a primer set of pgsC-R and BamHI_PspoVG FW (SEQ ID NO: 34) were used to prepare a 2.3 kb DNA fragment (P_spoVG/pgsBC) having a *Bacillus subtilis* spoVG gene promoter upstream of the *Bacillus subtilis pgsBC* gene. In addition, the thus-prepared DNA fragment was cloned into a plasmid vector in the same manner as in the Preparation Example 1, and the recombinant vector for producing PGA obtained was designated as pHY-P_spoVG/pgsBC.

### [Preparation Example 3] Construction of vector (3)

In this Preparation Example, a method of cloning genes relating to synthesis of PGA is described, in a similar manner to the Preparation Example 2 (see Fig. 3).

A chromosome DNA, serving as a template, prepared from *Bacillus subtilis* 168 strain and a primer set of rapA FW (SEQ ID NO: 35) and P_rapA/Cm R (SEQ ID NO: 36) shown in Table 1 were used to prepare an upstream 0.5 kb DNA fragment (rapA-DW) for insertion of the chloramphenicol-resistant gene derived from the plasmid pC194 to a site approximately 0.5 kb upstream of *rapA* gene ORF. Similarly, a primer set of P_rapA/Cm F (SEQ ID NO: 37) and P_rapA RV (SEQ ID NO: 38) shown in Table 1 was used to prepare a downstream 0.5 kb DNA fragment (rapA-DW) (then, the primer P_rapA RV was designed to modify the initiation codon of the *rapA* gene ORF from ttg to atg).

Subsequently, the thus-prepared fragments (rapA-UP) and (rapA-DW), and the DNA fragment (comp_Cm) prepared in the Preparation Example 2, as templates, and a primer set of rapA FW and P_rapA RV were used to prepare a composite 1.9 kb DNA fragment (Cm-P_rapA) of the three fragments by SOE-PCR method. The thus-prepared DNA fragment was used to transform *Bacillus subtilis* 168 strain by the competent cell method, and a colony growing on a LBCm agar medium was separated as a transformant.

Then, a genome DNA extracted from the thus-prepared transformant, serving as a template, and a primer set of comp_Cm FW and P_rapA RV shown in Table 1 were used to prepare a 1.4 kb DNA fragment (P-rapA-Cm2) containing the chloramphenicol-resistant gene and a promoter region of the *rapA* gene by PCR. Separately, a primer set of comp_P-rapA R/BSpgsB atg FW (SEQ ID NO: 39) and pgsC FW and a genome DNA prepared from *Bacillus subtilis* 168 strain, as a template, were used to prepare a *pgsB* gene 1.2 kb DNA fragment (pgsB2) by PCR. Further, three fragments of the thus-prepared (pgsB2) and (P-rapA-Cm2) and the DNA fragment (pgsB-UP) in the Preparation Example 2, as templates, and a primer set of pgsC FW and pgsB RV were used to prepare a 3.5 kb DNA fragment by SOE-PCR.

Subsequently, the thus-prepared DNA fragment was used to transform *Bacillus subtilis* 168 strain by the competent cell method in the similar manner as in the Preparation Example 2 (hereinafter, the thus-obtained recombinant *Bacillus subtilis* is referred to as 1cp-P_rapA/pgsBCAcm strain). A genome DNA sample was prepared from the obtained transformant, and this genome DNA sample, as a template, and a primer set of pgsC-R and BamHI_PrapA FW (SEQ ID NO: 40) were used to prepare a 2.2 kb DNA fragment (P_rapA/pgsBC) having a *Bacillus subtilis rapA* gene promoter upstream of the *Bacillus subtilis pgsBC* gene. Then, the thus-prepared DNA fragment was cloned into a plasmid vector in the same manner as in the Preparation Example 1, and the recombinant plasmid obtained was designated as recombinant vector for producing PGA pHY-P_rapA/pgsBC.

### [Preparation Example 4] Construction of vector (4)

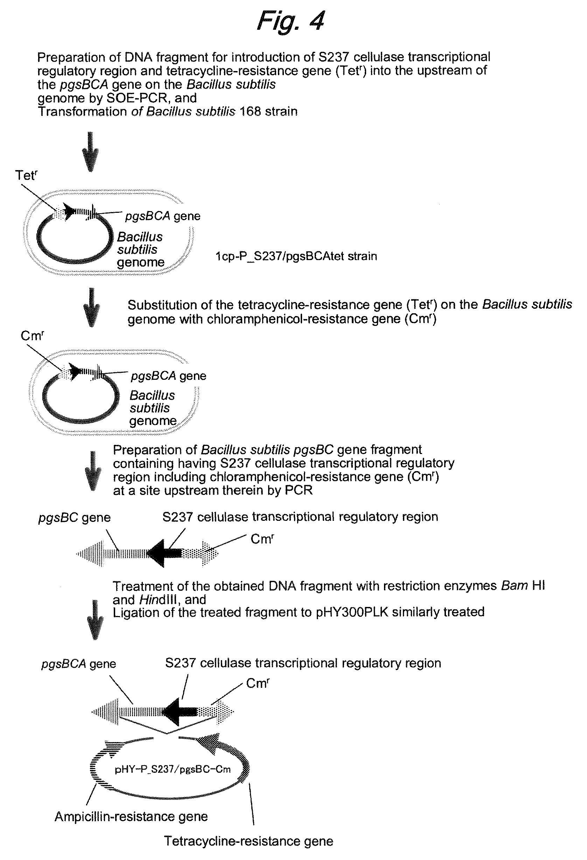

In this Preparation Example, a method of cloning genes relating to synthesis of PGA is described, in a similar manner to the Preparation Example 2 (see Fig. 4).

The pHY-P_S237/pgsBCA prepared in the Preparation Example 1, serving as a template, and a primer set of pgsC-R and tet4-1 (SEQ ID NO: 41) shown in Table 1 were used to prepare a 3.3 kb DNA fragment of *pgsB* gene (tet-P_B) containing a tetracycline resistance gene (Tet^{r}) on the plasmid, and an S237 cellulase promoter sequence in the upstream of the fragment. Separately, a chromosome DNA, serving as a template, prepared from *Bacillus subtilis* 168 strain and a primer set of tet4-1_comp/pgsB FW (SEQ ID NO: 42) and pgsB RV were used to prepare a 1.0 kb DNA fragment (pgsB-DW1) flanking the upstream of the *pgsBCA* gene on the *Bacillus subtilis* genome.

These fragments (tet-P_B) and (pgsB-DW1), as templates, and a primer set of pgsC-R and pgsB RV were used to bind the two fragments to each other by SOE-PCR method and to prepare a DNA fragment (tet-P_S237) for substitution of the promoter flanking the upstream of the *pgsBCA* gene operon of the *Bacillus subtilis* genome. Further, the obtained DNA fragment (tet-P_S237) was used to transform *Bacillus subtilis* 168 strain by the competent method, and a colony growing on a LBTc agar medium was separated as a transformant. The genome DNA sample was extracted from the transformant obtained, and PCR by using this sample as a template revealed that the transformant was a mutant *Bacillus subtilis* strain (hereinafter, referred to as 1cp-P_S237/pgsBCAtet strain) having a S237 cellulase promoter flanking the upstream of the *pgsBCA* gene operon.

Then, a chromosome DNA prepared from *Bacillus* sp. KSM-S237 strain (FERM BP-7875), serving as a template, and a primer set of P_S237-RV and P_S237-F were used to prepare an approximately 0.6 kb DNA fragment of the S237 cellulase promoter region (PS237-Cm) by PCR method. Separately, the plasmid pC194, as a template, and a primer set of P_S237_comp/Cm FW (SEQ ID NO: 43) and CmRV (SEQ ID NO: 23) were used to prepare a 0.9 kb DNA fragment of the chloramphenicol-resistant gene (Cm-PS237).

Subsequently, a 3.2 kb DNA fragment was obtained by SOE-PCR by using a mixture of the thus-prepared DNA fragments (PS237-Cm) and (Cm-PS237) and the DNA fragment (BCA-UP) prepared in Preparation Example 6, as templates, and a primer set of P_S237-RV and pgsB RV. The thus-prepared DNA fragment was used to transform a mutant *Bacillus subtilis* strain (1cp-P_S237/pgsBCAtet) by the competent cell method, and a colony growing on a LBCm agar medium was separated as a transformant.

Then, a chromosome DNA prepared from the thus-prepared transformant, serving as a template, and a primer set of pgsC-R and pC194CmRV/HindIII RV (SEQ ID NO: 44) were used to prepare a 3.4 kb *pgsBC* gene fragment containing a chloramphenicol-resistant gene and having S237 cellulase promoter sequence upstream thereof. After purification and recovery, the DNA fragment obtained was treated with a restriction enzyme *Hin*dIII, and ligated with (bound to) a plasmid vector pHY300PLK previously treated with the same restriction enzyme by using a DNA Ligation kit Ver.2. The above-described ligation sample was used to transform a *Bacillus subtilis* 168 strain by protoplast method, and a colony growing on a protoplast regeneration medium (DM3 medium) containing 5 ppm of chloramphenicol was collected as a transformant. The transformant obtained was grown once again on the LBCm agar medium, a plasmid was prepared from the microbe obtained by using High Pure Plasmid Isolation Kit, and the plasmid obtained was designated as a recombinant vector for producing PGA pHY-P_S237/pgsBC-Cm.

### [Preparation Example 5] Construction of Bacillus subtilis Mutant Strain (1)

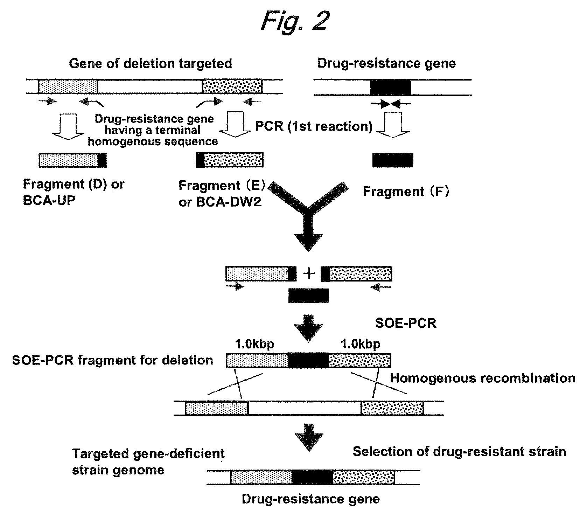

In this Preparation Example, a method of producing a *Bacillus subtilis* mutant strain for introduction of the vector obtained in the Preparation Example 1 is described (see Fig. 2).

First, a genome DNA prepared from *Bacillus subtilis* 168 strain, serving as a template, and any one of a primer set of ggt-F (SEQ ID NO: 18) and ggt/Cm-R (SEQ ID NO: 19) and a primer set of ggt/Cm-F (SEQ ID NO: 20) and ggt-R (SEQ ID NO: 21) shown in Table 1 were used to prepare a 1.0 kb DNA fragment (D) in contact with the upstream of the *ggt* gene on the genome and a 1.0 kb DNA fragment (E) in contact with the downstream of the *ggt* gene on the genome. Separately, the plasmid pC194, as a template, and a primer set of CmFW (SEQ ID NO: 22) and CmRV shown in Table 1 were used to prepare a 0.9 kb DNA fragment (F) containing a chloramphenicol-resistant gene.

Subsequently, the thus-prepared three fragments (D), (E) and (F) were mixed. Then, the thus-obtained template and a primer set of ggt-F and ggt-R were used to bind the three fragments each other in the order of (D)-(F)-(E) by SOE-PCR, to prepare a 2.9 kb DNA fragment for gene deletion. The thus-prepared DNA fragment was used to transform a *Bacillus subtilis* 168 strain by competent cell method, and a colony growing on a LBCm agar medium was separated as a transformant. Further, a genome DNA was extracted from the transformant obtained, and it was confirmed by the PCR using the DNA as a template that the transformant was a desirable *Bacillus subtilis* mutant strain (hereinafter, referred to as Δggt strain) in which the structural gene (ORF) of the *ggt* gene was replaced with the chloramphenicol-resistant gene (fragment F).

### [Preparation Example 6] Construction of Bacillus subtilis Mutant Strain (2)

In this Preparation Example, a method of producing a *Bacillus subtilis* mutant strain for introduction of the vector obtained in the Preparation Example 1 is described (see Fig. 2).

First, a genome DNA prepared from *Bacillus subtilis* 168 strain, serving as a template, and any one of a primer set of pgsA FW (SEQ ID NO: 45) and pgsA/Cm R (SEQ ID NO: 46) a primer set of pgsB/Cm F and pgsB RV shown in Table 1 were used to prepare a 1.0 kb DNA fragment (BCA-UP) in contact with the downstream of the *pgsBCA* gene on the genome and a 1.0 kb DNA fragment (BCA-DW2) in contact with the upstream of the *pgsBCA* gene on the genome.

Subsequently, the thus-prepared fragments (BCA-UP) and (BCA-DW2) and the DNA fragment (F) prepared in Preparation Example 5 were mixed to prepare a template. Then, the template and a primer set of pgsA FW and pgsB RV were used to bind the three fragments each other in the order of (BCA-UP)-(F)-(BCA-DW2) by SOE-PCR, to prepare a 2.9 kb DNA fragment for gene deletion. The thus-prepared DNA fragment for gene deletion was used to transform a *Bacillus subtilis* 168 strain by competent cell method, and a colony growing on a LBCm agar medium was separated as a transformant. Further, a genome DNA was extracted from the transformant obtained, and it was confirmed by the PCR using the DNA as a template that the transformant was a desirable *Bacillus subtilis* mutant strain (hereinafter, referred to as ΔBCA strain) in which the *pgsBCA* gene operon was replaced with the chloramphenicol-resistant gene (fragment F).

### [Preparation Example 7] Transformation by Introduction of Plasmid

In this Preparation Example, a method of transforming the *Bacillus subtilis* strain and the mutant strains thereof and other *Bacillus* microbes, as host cells.

*Bacillus subtilis* strains (*Bacillus subtilis* Marburg No. 168 strain, NCIMB11623 strain and IFO3215 strain), the *Bacillus subtilis* mutant strain prepared in Preparation Example 5 (Δggt strain), the *Bacillus subtilis* mutant strain prepared in Preparation Example 6 (ΔBCA strain), the 1cp-P_S237/pgsBCAcm strain prepared in Preparation Example 4, the 1cp-P_rapA/pgsBCAcm strain prepared in Preparation Example 3, a *Bacillus megaterium* ATCC14581 strain, and a *Bacillus megaterium* WH320 strain were transformed respectively with the recombinant vectors for producing PGA (pHY-P_S237/pgsBCA, pHY-P_S237/pgsBC, pHY-P_spoVG/pgsBC and pHY-P_rapA/pgsBC) obtained in Preparation Examples 1, 2 and 3 and a control vector (pHY300PLK). The wild strains and the mutant strains were treated with lysozyme, to prepare 10⁵ to 10⁶ protoplasts. To thus-obtained protoplasts, 20 to 100 ng of the plasmid DNA were added for introduction of the plasmid. A colony growing on a DM3 protoplast regeneration medium (DM3 medium) containing 20 ppm tetracycline hydrochloride salt was collected as a desirable transformant *Bacillus subtilis* strain containing the plasmid.

The *Bacillus licheniformis* ATCC9945a strain was transformed by using the recombinant vector for producing PGA obtained in Preparation Example 4 (pHY-P_S237/pgsBC-Cm), and a colony growing on a DM3 protoplast regeneration medium (DM3 medium) containing 15 ppm of chloramphenicol was collected as a desired plasmid-incorporated transformant.

### [Example 1] Evaluation of Productivity (1)

The transformant prepared in Example 7, in which pHY-P_S237/pgsBCA or pHY-P_S237/pgsBC was introduced into the *Bacillus subtilis* 168 strain, was inoculated on a LBTc agar medium and cultured stationarily at 30°C overnight. The *Bacillus subtilis* transformant growing on the LBTc agar medium was agitated and suspended in 0.8 to 1.6 mL of modified 2xL/Maltose medium (2.0% triptone, 1.0% yeast extract, 1.0% sodium chloride, 7.5% maltose, 7.5 ppm manganese sulfate tatra- or penta-hydrate, 15 ppm tetracycline hydrochloride salt) placed in a 2.0 mL small-capacity centrifuge tube previously sterilized, and the resulting supernatant after left still was used as seed culture liquid. The seed culture liquid was inoculated in a new modified 2xL/Maltose medium at 1% (v/v), and the medium was cultured in a Sakaguchi flask at 37°C while shaken reciprocally at 120 rpm for 3 days (on TB-20R-3F, manufactured by Takasaki Scientific Instrument). After test culture, the amount of produced PGA was determined under the analytical conditions shown in the following measurement example and the evaluation results are summarized in Table 2.

**Table 2**

| Host cell | Vector introduced | Amount of produced PGA | | |
|---|---|---|---|---|
| | | Culture for one day | Culture for two days | Culture for three days |
| 168 strain | pHY-P_S237/pgsBC | 4.64 | 1.58 | 0.08 |
| | pHY-P_S237/pgsBCA | ND | ND | ND |

As shown in Table 2, the strain containing the introduced *pgsBC* gene had PGA productivity higher than that of the control strain containing the *pgsBCA* gene.

### [Example 2] Evaluation of Productivity (2)

The transformant prepared in Preparation Example 7, in which pHY-P_S237/pgsBCA or pHY-P_S237/pgsBC was introduced into the *Bacillus subtilis* 168 strain, was inoculated on a LBTc agar medium and cultured stationarily at 30°C overnight. The *Bacillus subtilis* transformant was agitated and suspended in a modified 2xL/Maltose+MSG medium (2.0% triptone, 1.0% yeast extract, 1.0% sodium chloride, 7.5% maltose, 8.0% sodium glutamate monohydrate, 7.5 ppm manganese sulfate tatra- or penta-hydrate, and 15 ppm tetracycline hydrochloride salt), and the resulting supernatant after left still was used as seed culture liquid. The seed culture liquid was inoculated in a new modified 2xL/Maltose+MSG medium at 1% (v/v), and the medium was cultured in a Sakaguchi flask at 37°C while shaken reciprocally at 120 rpm for 3 days (on TB-20R-3F, Takasaki Scientific Instrument). After test culture, the amount of produced PGA was determined under the analytical conditions shown in the following measurement example and the evaluation results are summarized in Table 3.

**Table 3**

| Host cell | Vector introduced | Amount of produced PGA | | |
|---|---|---|---|---|
| | | Culture for one day | Culture for two days | Culture for three days |
| 168 strain | pHY-P_S237/pgsBC | 30.8 | 28.1 | 30.8 |
| | pHY-P_S237/pgsBCA | 0.75 | 1.38 | 1.19 |

As shown in Table 3, the PGA productivity was increased particularly significantly when there was an excessive amount of glutamic acid contained in the medium. It was also found that the PGA productivity remained high even after culture for three days when there was an excessive amount of glutamic acid in the medium.

### [Example 3] Evaluation of Productivity (3)

The PGA productivity of each of the *Bacillus subtilis* 168 strain, and the transformant prepared in Preparation Example 5, in which pHY-P_S237/pgsBC was introduced into the *Bacillus subtilis* mutant strain (Δ*ggt* strain) prepared in Preparation Example 5, was evaluated in a similar manner to Example 1. After test culture, the amount of produced PGA was determined under the analytical conditions shown in the following measurement example and the evaluation results are summarized in Table 4.

**Table 4**

| Host cell | Vector introduced | Amount of produced PGA | | |
|---|---|---|---|---|
| | | Culture for one day | Culture for two days | Culture for three days |
| 168 strain | pHY-P_S237/pgsBC | 4.67 | 3.64 | 1.71 |
| Δ*ggt* strain | pHY-P_S237/pgsBC | 5.05 | 5.56 | 5.25 |

As shown in Table 4, the Δ*ggt* strain, when used as the host, was superior in PGA productivity to the wild strain *Bacillus subtilis* 168 strain, and the PGA productivity remained high even after culture for three days.

### [Example 4] Evaluation of Productivity (4)

The PGA productivity of each of the *Bacillus subtilis* 168 strain, and the transformant prepared in Preparation Example 7, in which pHY-P_S237/pgsBC was introduced into the *Bacillus subtilis* mutant strain (Δ*BCA* strain) prepared in Preparation Example 6, was evaluated in a similar manner to Example 1. After test culture, the amount of produced PGA was determined under the analytical conditions shown in the following measurement example and the evaluation results are summarized in Table 5.

**Table 5**

| Host cell | Vector introduced | Amount of produced PGA | | |
|---|---|---|---|---|
| | | Culture for one day | Culture for two days | Culture for three days |
| 168 strain | pHY-P_S237/pgsBC | 4.41 | 3.11 | ND |
| ΔBCA strain | pHY-P_S237/pgsBC | 4.76 | 3.38 | ND |
| ΔBCA strain | pHY300PLK | ND | ND | ND |

As shown in Table 5, not only the *Bacillus subtilis* 168 strain having *pgsBCA* gene on its chromosome but no ability of producing PGA, but also the Δ*BCA* strain deficient in the *pgsBCA* gene showed high PGA productivity.

### [Example 5] Evaluation of productivity (5)

The PGA productivity of the transformant prepared in Example 7, in which pHY-P_S237/pgsBC, pHY-P_spoVG/pgsBC or pHY-P_rapA/pgsBC was introduced into *Bacillus subtilis* 168 strain, was evaluated in a similar manner to Example 2. After test culture, the amount of produced PGA was determined under the analytical conditions shown in the following measurement example and the evaluation results are summarized in Table 6.

**Table 6**

| Host cell | Vector introduced | Amount of produced PGA | | |
|---|---|---|---|---|
| | | Culture for one day | Culture for two days | Culture for three days |
| 168 strain | pHY-P_S237/pgsBC | | | 43.1 |
| | pHY-P_spoVG/pgsBC | | | 10.2 |
| | pHY-P_rapA/pgsBC | | | 67.3 |

As shown in Table 6, by using the *rapA* gene promoter and the *spoVG* gene derived from *Bacillus subtilis,* as well as the S237 cellulase promoter, the *Bacillus subtilis* transformant containing the *pgsBC* gene-containing recombinant vector for producing PGA showed high PGA productivity.

### [Example 6] Evaluation of Productivity (6)

The PGA productivity of each of the transformants prepared in Preparation Example 7, in which pHY-P_S237/pgsBC was introduced into the *Bacillus subtilis* strain NCIMB11623 and IFO3215, was evaluated in a similar manner to Example 2. After test culture, the amount of produced PGA was determined under the analytical conditions shown in the following measurement example and the evaluation results are summarized in Table 7.

**Table 7**

| Host cell | Vector introduced | Amount of produced PGA | | |
|---|---|---|---|---|
| | | Culture for one day | Culture for two days | Culture for three days |
| 168 strain | pHY-P_S237/pgsBC | | | 35.5 |
| NCIMB11623 strain | pHY-P_S237/pgsBC | | | 25.0 |
| | pHY300PLK | | | ND |
| IFO3215 strain | pHY-P_S237/pgsBC | | | 11.5 |
| | pHY300PLK | | | ND |

As shown in Table 7, introduction of the recombinant vector for producing PGA containing only the *pgsBC* gene to *Bacillus subtilis* 168 strain as well as other *Bacillus subtilis* species was effective in providing PGA productivity.

### [Example 7] Evaluation of Productivity (7)

The PGA productivity of the transformant prepared in Example 7, in which pHY-P_S237/pgsBC-Cm was introduced into *Bacillus licheniformis* ATCC9945a strain, was evaluated in a similar manner to Example 2 (in this case, as the antibiotic to be used, 15-ppm tetracycline was changed to 5-ppm chloramphenicol). After test culture, the amount of produced PGA was determined under the analytical conditions shown in the following measurement example and the evaluation results are summarized in Table 8.

**Table 8**

| Host cell | Vector introduced | Amount of produced PGA | | |
|---|---|---|---|---|
| | | Culture for one day | Culture for two days | Culture for three days |
| ATCC9945a | No plasmid introduced | | | 0.96 |
| | pHY-P_S237/pgsBC-Cm | | | 3.00 |

As shown in Table 8, introduction of recombinant vector for producing PGA containing the *pgsBC* gene to, as well as *Bacillus subtilis,* other *Bacillus* microbes was effective in increasing the PGA productivity.

### [Example 8] Evaluation of Productivity (8)

The PGA productivity of each of the transformant prepared in Preparation Example 7, in which pHY-P_spoVG/pgsBC was introduced into *Bacillus megaterium* ATCC14581 strain, and the transformant prepared in Preparation Example 7, in which pHY-P_S237/pgsBC was introduced into *Bacillus megaterium* WH320 strain, was evaluated in a similar manner to Example 2. After test culture, the amount of produced PGA was determined under the analytical conditions shown in the following measurement example and the evaluation results are summarized in Table 9.

**Table 9**

| Host cell | Vector introduced | Amount of produced PGA | | |
|---|---|---|---|---|
| | | Culture for one day | Culture for two days | Culture for three days |
| ATCC14581 strain | pHY300PLK | | | 0.05 |
| | pHY-P_spoVG/pgsBC | | | 0.55 |
| WH320 strain | pHY300PLK | | | 5.05 |
| | pHY-P_S237/pgsBC | | | 6.98 |

As shown in Table 9, introduction of recombinant vector for producing PGA containing the *pgsBC* gene to, as well as *Bacillus subtilis,* other *Bacillus* microbes was effective in increasing the PGA productivity.

### [Example 9] Evaluation of Productivity (9) (Molecular Weight Evaluation)

The PGA productivity employing each of the transformant prepared in Preparation Example 7, in which pHY-P_S237/pgsBC was introduced into the 1cp-P_rapA/pgsBCAcm strain and the 1cp-P_S237/pgsBCAcm strain prepared in Preparation Examples 3 and 4, was evaluated in a similar manner to Example 2. After test culture, the amount of PGA produced and the molecular weight thereof were determined under the analytical conditions shown in the following measurement example and the evaluation results are summarized in Tables 10 and 11.

**Table 10**

| Host cell | Vector introduced | Amount of produced PGA | | |
|---|---|---|---|---|
| | | Culture for one day | Culture for two days | Culture for three days |
| 1cp-P_S237/ pgsBCAcm | pHY300PLK | | | 28.9 |
| | pHY-P_S237/pgsBC | | | 39.4 |
| 1 cp-P_rapA/ pgsBCAcm | pHY300PLK | | | 3.2 |
| | pHY-P_S237/pgsBC | | | 33.2 |

As shown in Table 10, introduction of a recombinant vector for producing PGA having only the *pgsBC* gene into the *Bacillus subtilis* mutant capable of producing PGA was found to increase PGA productivity, compared to the strain without introduction of the gene.

**Table 11**

| Host cell | Vector introduced | Molecular weight of PGA | |
|---|---|---|---|
| | | Culture for one day | Culture for three days |
| 1cp-P_S237/ pgsBCAcm | pHY300PLK | 7,000,000 to 8,500,000 | 6,300,000 to 7,600,000 |
| | pHY-P_S237/ pgsBC | 7,500,000 to 10,400,000 | 6,200,000 to 9,100,000 |
| 1 cp-P_rapA/ pgsBCAcm | pHY300PLK | 340,000 to 860,000 | 350,000 to 830,000 |
| | pHY-P_S237/ pgsBC | 6,900,000 to 12,400,000 | 5,900,000 to 8,100,000 |

As shown in Table 11, introduction of a recombinant vector for producing PGA having only the *pgsBC* gene into the *Bacillus subtilis* mutant capable of producing PGA was found to increase the molecular weight of the PGA produced, compared to the strain without introduction of the gene.

[Measurement Example] Method of quantitatively determining amount of PGA produced and measuring the molecular weight of PGA produced.

Each of the culture samples after test culture shown in Examples 1 to 9 was centrifuged at room temperature at 14,800 rpm for 30 minutes (trade name: himac CF15RX, Hitachi Koki), and the amount of the PGA produced by using the recombinant microorganisms in the culture supernatant obtained after centrifugation was determined. PGA in the supernatant sample was analyzed according to the method by Yamaguchi et al. (described above). Specifically, PGA production by using the recombinant microorganisms was confirmed by subjecting the sample to agarose gel electrophoresis, staining the gel with methylene blue, and observing the presence of stained substances derived from PGA. The sample containing PGA produced by the recombinant microorganisms thus detected was analyzed by HPLC using TSKGel G4000PWXL and TSKGel G6000PWXL gel filtration columns (trade name, Tosoh Corporation). As for the analytical condition, the eluate used was 0.1 M sodium sulfate, the flow rate was 1.0 mL /minute, the column temperature was 50°C, and the UV detection wavelength was 210 nm. In determination of the concentration, a PGA having a molecular weight of 800k (Meiji Food Materia) was used for preparation of a calibration curve. For determination of the molecular weight, various poly-glutamic acid samples having different molecular weights [Wako Pure Chemical Industries (162-21411, 162-21401), SIGMA-ALDRICH (P-4886, P-4761), Meiji Food Materia (molecular weight: 880k)], which were previously determined by using pullulan (Shodex STANDRD P-82, trade name, Showa Denko K.K.), were used as standards. The unit of the values showing the PGA-productivity in the Tables showing the evaluation results, is (g/L). The notation "ND" in the Tables showing the evaluation results, means that the amount of PGA produced did not attain the detection limit.

### [Example 10] Evaluation of productivity (10) (Measurement of Optical Purity of PGA Composition)

The culture solution obtained from the *Bacillus subtilis* 168 strain transformant having pHY-P_S237/pgsBCA introduced in Example 2, and the culture solution obtained the transformant of the *Bacillus subtilis* mutant strain (Δ*BCA* strain) having pHY-P_S237/pgsBC introduced in Example 4 were used as samples for measurement of optical purity. An equivalent amount of ethanol was added to the supernatant of the culture, and the precipitates generated were collected by centrifugation. Subsequently, the collected precipitates were subjected to dryness, and the resultant was encapsulated with 6N hydrochloric acid under nitrogen and heat-treated at 115°C to 120°C for 24 hours. After the heat treatment, hydrochloric acid and water were removed by distillation under nitrogen gas flow, to give a hydrolysate sample. Subsequently, the amino acid composition and the L-glutamic acid content in the hydrolysate sample were analyzed quantitatively by using a full automatic amino acid analyzer L-8500 (trade name, Hitachi Instrument Service) and L-glutamic acid measurement kit (Yamasa Corp). The analysis by using the full automatic amino acid spectrometer gave the total content of the optically active isomers (D- and L-isomers) quantitatively, and the D-isomer content was calculated by subtracting the quantitative content obtained by the L-isomer measurement kit from the total content. The PGA prepared by the *Bacillus* sp. KSM-366 strain (FERM BP-6262) was used as the PGA sample produced by wild-type *Bacillus* microbe. For evaluation of the racemization rate of the amino acid during the hydrolytic reaction, D- and L-glutamic acids (Wako Pure Chemical Industries) were used as standard samples. The results are summarized in Table 12.

**Table 12**

| Sample | Glutamic acid composition | |
|---|---|---|
| | D-isomer | L-isomer |
| Supernatant of Culture of 168 strain containing pHY-P_S237/pgsBCA introduced | 75 | 25 |
| Supernatant of Culture of ΔBCA strain containing pHY-P_S237/pgsBC introduced | 6 | 94 |
| PGA produced by *Bacillus* sp. KSM-366 strain | 79 | 21 |
| L-Glutamic acid (reagent: for evaluation of racemization rate) | 7 | 93 |
| D-Glutamic acid (reagent: for evaluation of racemization rate) | 91 | 9 |

As shown in Table 12, the recombinant *Bacillus subtilis* containing the introduced recombinant vector for producing PGA having only the *pgsBC* gene produced a glutamic acid at a favorably high optical purity of L-glutamic acid content of 90% or more. The racemization rate of an amino acid under the hydrolytic condition separately analyzed was approximately 10%. From these facts, it was indicated that the optical purity of the PGA obtained by the recombinant *Bacillus subtilis* containing only the *pgsBC* gene introduced was 80% to 100%.

### [Reference Example]

In a similar manner to Preparation Example 1, a chromosome DNA, serving as a template, prepared from *Bacillus* sp. KSM-366 stain (FERM BP-6262) and a primer set of pgsB-F and pgsB-R (SEQ ID NO: 15) shown in Table 1 were used to prepare a 1.2 kb DNA fragment (G) of the *pgsB* gene. Separately, the DNA fragment (C) of the cellulase gene promoter region derived from KSM-S237 strain, serving as a template, and a primer set of P_S237-FW and pgsB-R were used to prepare a composite 1.8 kb DNA fragment (GC) of fragments (G) and (C) by SOE-PCR method. A recombinant plasmid containing the DNA fragment (GC) inserted between the *Bam*HI and *Hin*dIII restriction enzyme recognition sites of plasmid vector pHY300PLK (Takara) was prepared then by an experimental method similar to that shown in Preparation Example 1, and was designated as recombinant vector for producing PGA pHY-P_S237/pgsB.

Using pHY300PLK having no PGA production gene introduced and the plasmid expressing only the *pgsB* gene (pHY-P_S237/pgsB) described above, the PGA productivity was determined by the methods described in Example 2 and 3. As a result, in both cases, it was confirmed that no PGA was produced.

### INDUSTRIAL APPLICABILITY

The recombinant microorganism according to the present invention has ability of producing poly-γ-glutamic acid. Thus, the recombinant microorganism according to the present invention can be used preferably in production of poly-γ-glutamic acid. It is also possible to produce a poly-γ-glutamic acid having desired molecular weight and structure, by employing the recombinant microorganism according to the present invention. Thus, the recombinant microorganism according to the present invention can be used preferably for improvement in productivity of the poly-γ-glutamic acid, and adjusting molecular weight and optical purity of the poly-γ-glutamic acid.

This application claims priority on Patent Application No. 2007-244023 filed in Japan on September 20, 2007.

## Claims

1. A recombinant microorganism having ability of producing poly-γ-glutamic acid, wherein, the recombinant microorganism is being prepared by introducing a *Bacillus subtilis pgsB* gene, and a *Bacillus subtilis pgsC* gene into a host microorganism, wherein no *Bacillus subtilis pgsA* gene or a gene having a nucleotide sequence with a genetic identity to the nucleotide sequence set forth in SEQ ID NO:5 of 80% or more and coding a protein having been considered to have PGA synthesis activity together with PgsB and PgsC, is being introduced into the host microorganism, wherein the host microorganism is a *Bacillus* microbe selected from *Bacillus subtilis, Bacillus licheniformis* and *Bacillus megaterium,* wherein the *pgsB* gene and the *pgsC* gene are incorporated in a plasmid self-replicable in a cell of the host microorganism and wherein multiple copies of said plasmid are contained in the cell.

2. The recombinant microorganism according to Claim 1, wherein the *pgsB* gene is a gene coding the following protein (a) or (b):
(a) a protein having the amino acid sequence set forth in SEQ ID NO: 2;
(b) a protein having an amino acid sequence, in which 2 to 80 amino acids are being deleted, substituted, added or inserted in the amino acid sequence set forth in SEQ ID NO: 2, and having amido-ligase activity.

3. The recombinant microorganism according to Claim 1, wherein the *pgsB* gene is a gene having a nucleotide sequence with a genetic identity to the nucleotide sequence set forth in SEQ ID NO: 1 of 80% or more and coding a protein having amido-ligase activity.

4. The recombinant microorganism according to Claim 1 to 3, wherein the *pgsC* gene is a gene coding the following protein (c) or (d):
(c) a protein having the amino acid sequence set forth in SEQ ID NO: 4; and
(d) a protein having an amino acid sequence, in which 2 to 30 amino acids are being deleted, substituted, added or inserted in the amino acid sequence set forth in SEQ ID NO: 4, and having a function of producing poly-γ-glutamic acid in the presence of the PgsB protein coded by the *pgsB* gene.

5. The recombinant microorganism according to Claim 1 to 3, wherein the *pgsC* gene is a gene having a nucleotide sequence with a genetic identity to the nucleotide sequence set forth in SEQ ID NO:3 of 80% or more and coding a protein having ability of producing PGA in the presence of the PgsB protein.

6. The recombinant microorganism according to any one of Claims 1 to 5, wherein the *pgsB* gene, and the *pgsC* gene, have a transcription initiation regulatory region and/or a translation initiation regulatory region each functioning in the microorganism at a particular site upstream of the genes.

7. The recombinant microorganism according to any one of Claims 1 to 6, wherein the *Bacillus* microbe is *Bacillus subtilis.*

8. The recombinant microorganism according to Claim 7, wherein the *Bacillus subtilis* is *Bacillus subtilis* Marburg No. 168 strain.

9. A method of producing a poly-γ-glutamic acid, comprising the steps of:
culturing the recombinant microorganism according to any one of Claims 1 to 8, and
collecting poly-γ-glutamic acid produced.

10. A method of improving a productivity in poly-γ-glutamic acid production or adjusting molecular weight of poly-γ-glutamic acid, which comprises employing a recombinant microorganism, which is obtained by introducing a *Bacillus subtilis pgsB* gene and a *Bacillus subtilis pgsC* gene into a host microorganism, wherein the host microorganism is a *Bacillus* microbe selected from *Bacillus subtilis, Bacillus licheniformis* and *Bacillus megaterium.*

11. A method of adjusting optical purity of poly-γ-glutamic acid, wherein L-isomer ratio of the poly-γ-glutamic acid produced is controlled by employing a recombinant microorganism, which is obtained by introducing a *Bacillus subtilis pgsB* gene, and a *Bacillus subtilis pgsC* gene into a host microorganism, wherein the host microorganism is a *Bacillus* microbe selected from *Bacillus subtilis, Bacillus licheniformis* and *Bacillus megaterium.*

12. The method of adjusting optical purity of poly-γ-glutamic acid according to Claim 11 or the method of improving a productivity in poly-γ-glutamic acid production or adjusting molecular weight of poly-γ-glutamic acid according to claim 10, wherein the recombinant microorganism is the recombinant microorganism according to any one of Claims 1 to 8.

13. A method of producing a high-molecular-weight poly-γ-glutamic acid, which comprises employing the method of adjusting molecular weight of poly-γ-glutamic acid according to Claim 10 or 12.

14. A method of producing poly-γ-glutamic acid having high L-isomer ratio, which comprises employing the method of adjusting optical purity of poly-γ-glutamic acid according to Claim 11 or 12.

15. Use of the recombinant microorganism according to any one of Claims 1 to 8.

## Patentansprüche

1. Rekombinanter Mikroorganismus, der die Fähigkeit hat, Poly-γ-Glutaminsäure herzustellen, wobei der rekombinante Mikroorganismus durch das Einführen eines *Bacillus subtilis pgsB*-Gens und eines *Bacillus subtilis* pgsC-Gens in einen Wirtsmikroorganismus hergestellt wird, wobei kein *Bacillus subtilis pgsA-*Gen oder ein Gen, das eine Nucleotidsequenz mit einer genetischen Identität von 80% oder höher zu der in SEQ ID NO:5 gezeigten Nucleotidsequenz aufweist und das ein Protein codiert, von dem angenommen wird, dass es zusammen mit PgsB und PgsC PGA-Syntheseaktivität hat, in den Wirtsmikroorganismus eingeführt wird, wobei der Wirtsmikroorganismus eine *Bacillus*-Mikrobe ist, die ausgewählt ist aus *Bacillus subtilis, Bacillus licheniformis* und *Bacillus megaterium,* wobei das *pgsB*-Gen und das *pgsC-*Gen in einem Plasmid integriert sind, das sich in einer Zelle des Wirtsmikroorganismus selbst replizieren kann, und wobei mehrere Kopien des Plasmids in der Zelle enthalten sind.

2. Rekombinanter Mikroorganismus nach Anspruch 1, wobei das *pgsB*-Gen ein Gen ist, das das folgende Protein (a) oder (b) codiert:
(a) ein Protein, das die in SEQ ID NO:2 gezeigte Aminosäuresequenz aufweist;
(b) ein Protein, das eine Aminosäuresequenz aufweist, in der 2 bis 80 Aminosäuren in der in SEQ ID NO:2 gezeigten Aminosäuresequenz deletiert, substituiert, hinzugefügt oder inseriert werden, und das Amidoligase-Aktivität aufweist.

3. Rekombinanter Mikroorganismus nach Anspruch 1, wobei das *pgsB*-Gen ein Gen ist, das eine Nucleotidsequenz mit einer genetischen Identität von 80% oder höher zu der in SEQ ID NO:1 gezeigten Nucleotidsequenz aufweist und das ein Protein codiert, das Amidoligase-Aktivität aufweist.

4. Rekombinanter Mikroorganismus nach den Ansprüchen 1 bis 3, wobei das *pgsC*-Gen ein Gen ist, das die folgenden Proteine (c) oder (d) codiert:
(c) ein Protein, das die in SEQ ID NO:4 gezeigte Aminosäuresequenz aufweist; und
(d) ein Protein, das eine Aminosäuresequenz aufweist, in der 2 bis 30 Aminosäuren in der in SEQ ID NO:4 gezeigten Aminosäuresequenz deletiert, substituiert, hinzugefügt oder inseriert werden und das eine Funktion hat, Poly-γ-Glutaminsäure in Anwesenheit des PgsB-Proteins, das von dem *pgsB*-Gen codiert wird, herzustellen.

5. Rekombinanter Mikroorganismus nach den Ansprüchen 1 bis 3, wobei das *pgsC*-Gen ein Gen ist, das eine Nucleotidsequenz mit einer genetischen Identität von 80% oder höher zu der in SEQ ID NO:3 gezeigten Nucleotidsequenz aufweist und das ein Protein codiert, das die Fähigkeit hat, PGA in Anwesenheit des PgsB-Proteins herzustellen.

6. Rekombinanter Mikroorganismus nach einem der Ansprüche 1 bis 5, wobei das *pgsB*-Gen und das *pgsC*-Gen eine regulatorische Initiationsregion der Transkription und/oder eine regulatorische Initiationsregion der Translation hat, wobei jede im Mikroorganismus an einer bestimmten Stelle stromaufwärts der Gene funktionell ist.

7. Rekombinanter Mikroorganismus nach einem der Ansprüche 1 bis 6, wobei die *Bacillus*-Mikrobe *Bacillus subtilis* ist.

8. Rekombinanter Mikroorganismus nach Anspruch 7, wobei der *Bacillus subtilis* der Stamm *Bacillus subtilis* Marburg Nr. 168 ist.

9. Verfahren zur Herstellung einer Poly-γ-Glutaminsäure, umfassend die Schritte:
Züchten des rekombinanten Mikroorganismus nach einem der Ansprüche 1 bis 8 und Gewinnen der hergestellten Poly-γ-Glutaminsäure.

10. Verfahren zur Verbesserung der Produktivität der Herstellung von Poly-γ-Glutaminsäure oder zur Anpassung des Molekulargewichts von Poly-γ-Glutaminsäure, umfassend das Verwenden eines rekombinanten Mikroorganismus, der durch das Einführen eines *Bacillus subtilis pgsB*-Gens und eines *Bacillus subtilis pgsC*-Gens in einen Wirtsmikroorganismus erhalten wird, wobei der Wirtsmikroorganismus eine *Bacillus*-Mikrobe ausgewählt aus *Bacillus subtilis, Bacillus licheniformis* und *Bacillus megaterium* ist.

11. Verfahren zur Anpassung der optischen Reinheit von Poly-γ-Glutaminsäure, wobei das L-Isomer-Verhältnis der hergestellten Poly-γ-Glutaminsäure durch die Verwendung eines rekombinanten Mikroorganismus gesteuert wird, der durch das Einführen eines *Bacillus subtilis pgsB*-Gens und eines *Bacillus subtilis pgsC*-Gens in einen Wirtsmikroorganismus erhalten wird, wobei der Wirtsmikroorganismus eine *Bacillus*-Mikrobe ausgewählt aus *Bacillus subtilis, Bacillus licheniformis* und *Bacillus megaterium* ist.

12. Verfahren zur Anpassung der optischen Reinheit von Poly-γ-Glutaminsäure nach Anspruch 11 oder Verfahren zur Verbesserung der Produktivität der Herstellung von Poly-γ-Glutaminsäure oder zur Anpassung des Molekulargewichts von Poly-γ-Glutaminsäure nach Anspruch 10, wobei der rekombinante Mikroorganismus der rekombinante Mikroorganismus nach einem der Ansprüche 1 bis 8 ist.

13. Verfahren zur Herstellung einer Poly-γ-Glutaminsäure mit hohem Molekulargewicht, umfassend die Verwendung des Verfahrens zur Anpassung des Molekulargewichts von Poly-γ-Glutaminsäure nach Anspruch 10 oder 12.

14. Verfahren zur Herstellung von Poly-γ-Glutaminsäure, die ein hohes L-Isomer-Verhältnis aufweist, umfassend die Verwendung des Verfahrens zur Anpassung der optischen Reinheit von Poly-γ-Glutaminsäure nach Anspruch 11 oder 12.

15. Verwendung des rekombinanten Mikroorganismus nach einem der Ansprüche 1 bis 8.

## Revendications

1. Micro-organisme recombinant ayant la capacité de produire un acide poly-γ-glutamique (PGA), où, le micro-organisme recombinant est préparé par introduction d'un gène *pgsB* de *Bacillus subtilis,* et d'un gène *pgsC* de *Bacillus subtilis* dans un micro-organisme hôte, où aucun gène *pgsA* de *Bacillus subtilis* ou un gène ayant une séquence nucléotidique présentant une identité génétique de 80 % ou plus avec la séquence nucléotidique représentée par SEQ ID NO : 5 et codant pour une protéine ayant été considérée comme étant dotée d'une activité de synthèse de PGA conjointement avec PgsB et PgsC, est introduit dans le micro-organisme hôte, où le micro-organisme hôte est un microbe *Bacillus* choisi parmi *Bacillus subtilis, Bacillus licheniformis* et *Bacillus megaterium,* où le gène *pgsB* et le gène *pgsC* sont incorporés dans un plasmide auto-réplicable dans une cellule du micro-organisme hôte et où de multiples copies dudit plasmide sont contenues dans la cellule.

2. Micro-organisme recombinant selon la revendication 1, dans lequel le gène *pgsB* est un gène codant pour la protéine (a) ou (b) suivante :
(a) une protéine ayant la séquence d'acides aminés représentée par SEQ ID NO : 2 ;
(b) une protéine ayant une séquence d'acides aminés, dans laquelle 2 à 80 acides aminés sont délétés, substitués, ajoutés ou insérés dans la séquence d'acides aminés représentée par SEQ ID NO : 2, et étant dotée d'une activité amido-ligase.

3. Micro-organisme recombinant selon la revendication 1, dans lequel le gène *pgsB* est un gène ayant une séquence nucléotidique présentant une identité génétique de 80 % ou plus avec la séquence nucléotidique représentée par SEQ ID NO : 1 et codant pour une protéine dotée d'une activité amido-ligase.

4. Micro-organisme recombinant selon les revendications 1 à 3, dans lequel le gène *pgsC* est un gène codant pour la protéine (c) ou (d) suivante :
(c) une protéine ayant la séquence d'acides aminés représentée par SEQ ID NO : 4 ; et
(d) une protéine ayant une séquence d'acides aminés, dans laquelle 2 à 30 acides aminés sont délétés, substitués, ajoutés ou insérés dans la séquence d'acides aminés représentée par SEQ ID NO : 4, et ayant une fonction de production d'acide poly-γ-glutamique en présence de la protéine PgsB codée par le gène *pgsB.*

5. Micro-organisme recombinant selon les revendications 1 à 3, dans lequel le gène *pgsC* est un gène ayant une séquence nucléotidique présentant une identité génétique de 80 % ou plus avec la séquence nucléotidique représentée par SEQ ID NO : 3 et codant pour une protéine ayant la capacité de produire du PGA en présence de la protéine PgsB.

6. Micro-organisme recombinant selon l'une quelconque des revendications 1 à 5, dans lequel le gène *pgsB,* et le gène *pgsC,* ont une région régulatrice d'initiation de la transcription et/ou une région régulatrice d'initiation de la traduction, chacune fonctionnant dans le micro-organisme au niveau d'un site particulier en amont des gènes.

7. Micro-organisme recombinant selon l'une quelconque des revendications 1 à 6, où le microbe *Bacillus* est *Bacillus subtilis.*

8. Micro-organisme recombinant selon la revendication 7, où le *Bacillus subtilis* est la souche de *Bacillus subtilis* Marburg No. 168.

9. Méthode de production d'un acide poly-γ-glutamique, comprenant les étapes suivantes :
la culture du micro-organisme recombinant selon l'une quelconque des revendications 1 à 8, et
la collecte de l'acide poly-γ-glutamique produit.

10. Méthode d'amélioration de la productivité de la production d'acide poly-γ-glutamique ou d'ajustement du poids moléculaire de l'acide poly-γ-glutamique, qui comprend l'utilisation d'un micro-organisme recombinant, qui est obtenu par l'introduction d'un gène *pgsB* de *Bacillus subtilis* et d'un gène *pgsC* de *Bacillus subtilis* dans un micro-organisme hôte, où le micro-organisme hôte est un microbe *Bacillus* choisi parmi *Bacillus subtilis, Bacillus licheniformis* et *Bacillus megaterium.*

11. Méthode d'ajustement de la pureté optique de l'acide poly-γ-glutamique, dans laquelle le rapport de l'isomère L de l'acide poly-γ-glutamique produit est contrôlé en utilisant un micro-organisme recombinant, qui est obtenu par l'introduction d'un gène *pgsB* de *Bacillus subtilis* et d'un gène *pgsC* de *Bacillus subtilis* dans un micro-organisme hôte, où le micro-organisme hôte est un microbe *Bacillus* choisi parmi *Bacillus subtilis, Bacillus licheniformis* et *Bacillus megaterium.*

12. Méthode d'ajustement de la pureté optique de l'acide poly-γ-glutamique selon la revendication 11 ou méthode d'amélioration de la productivité de la production d'acide poly-γ-glutamique ou d'ajustement du poids moléculaire de l'acide poly-γ-glutamique selon la revendication 10, dans laquelle le micro-organisme recombinant est le micro-organisme recombinant selon l'une quelconque des revendications 1 à 8.

13. Méthode de production d'un acide poly-γ-glutamique de haut poids moléculaire, qui comprend l'utilisation de la méthode d'ajustement du poids moléculaire de l'acide poly-γ-glutamique selon la revendication 10 ou 12.

14. Méthode de production d'un acide poly-γ-glutamique présentant un ratio élevé de l'isomère L, qui comprend l'utilisation de la méthode d'ajustement de la pureté optique de l'acide poly-γ-glutamique selon la revendication 11 ou 12.

15. Utilisation du micro-organisme recombinant selon l'une quelconque des revendications 1 à 8.


### SEQUENCE LISTING

<110> Kao Corporation
<120> A recombinant microorganism and a method for producing poly-gamma-gultamic acid
<130> S1424 EP S3
<140> EP 08 83 1819.1
   <141> 2008-09-19
<150> JP 2007-244023
   <151> 2007-09-20
<160> 52
<170> PatentIn version 3.4
<210> 1
   <211> 1182
   <212> DNA
   <213> Bacillus subtilis
<220>
   <221> CDS
   <222> (1)..(1182)
<400> 1
   atg tgg tta ctc att ata gcc tgt gct gtc ata ctg gtc atc gga ata      48
   Met Trp Leu Leu Ile Ile Ala Cys Ala Val Ile Leu Val Ile Gly Ile
   1               5                   10                  15
   tta gaa aaa cga cga cat cag aaa aac att gat gcc ctc cct gtt cgg      96
   Leu Glu Lys Arg Arg His Gln Lys Asn Ile Asp Ala Leu Pro Val Arg
               20                  25                  30
   gtg aat att aac ggc atc cgc gga aaa tcg act gtg aca agg ctg aca      144
   Val Asn Ile Asn Gly Ile Arg Gly Lys Ser Thr Val Thr Arg Leu Thr
           35                  40                  45
   acc gga ata tta ata gaa gcc ggt tac aag act gtt gga aaa aca aca      192
   Thr Gly Ile Leu Ile Glu Ala Gly Tyr Lys Thr Val Gly Lys Thr Thr
       50                  55                  60
   gga aca gat gca aga atg att tac tgg gac aca ccg gag gaa aag ccg      240
   Gly Thr Asp Ala Arg Met Ile Tyr Trp Asp Thr Pro Glu Glu Lys Pro
   65                  70                  75                  80
   att aaa cgg aaa cct cag ggg ccg aat atc gga gag caa aaa gaa gtc      288
   Ile Lys Arg Lys Pro Gln Gly Pro Asn Ile Gly Glu Gln Lys Glu Val
                   85                  90                  95
   atg aga gaa aca gta gaa aga ggg gct aac gcg att gtc agt gaa tgc      336
   Met Arg Glu Thr Val Glu Arg Gly Ala Asn Ala Ile Val Ser Glu Cys
               100                 105                 110
   atg gct gtt aac cca gat tat caa atc atc ttt cag gaa gaa ctt ctg      384
   Met Ala Val Asn Pro Asp Tyr Gln Ile Ile Phe Gln Glu Glu Leu Leu
           115                 120                 125
   cag gcc aat atc ggc gtc att gtg aat gtt ttg gaa gac cat atg gat      432
   Gln Ala Asn Ile Gly Val Ile Val Asn Val Leu Glu Asp His Met Asp
       130                 135                 140
   gtc atg ggg ccg acg ctt gat gaa att gca gaa gcg ttt act gct aca      480
   Val Met Gly Pro Thr Leu Asp Glu Ile Ala Glu Ala Phe Thr Ala Thr
   145                 150                 155                 160
   att cct tat aat ggc cat ctt gtc att aca gat agt gaa tat acc gag      528
   Ile Pro Tyr Asn Gly His Leu Val Ile Thr Asp Ser Glu Tyr Thr Glu
                   165                 170                 175
   ttc ttt aaa caa aaa gca aaa gaa cga aac aca aaa gtc atc att gct      576
   Phe Phe Lys Gln Lys Ala Lys Glu Arg Asn Thr Lys Val Ile Ile Ala
               180                 185                 190
   gat aac tca aaa att aca gat gag tat tta cgt aaa ttt gaa tac atg      624
   Asp Asn Ser Lys Ile Thr Asp Glu Tyr Leu Arg Lys Phe Glu Tyr Met
           195                 200                 205
   gta ttc cct gat aac gct tct ctg gcg ctg ggt gtg gct caa gca ctc      672
   Val Phe Pro Asp Asn Ala Ser Leu Ala Leu Gly Val Ala Gln Ala Leu
       210                 215                 220
   ggc att gac gaa gaa aca gca ttt aag gga atg ctg aat gcg ccg cca      720
   Gly Ile Asp Glu Glu Thr Ala Phe Lys Gly Met Leu Asn Ala Pro Pro
   225                 230                 235                 240
   gat ccg gga gca atg aga att ctt ccg ctg atc agt ccg agc gag cct      768
   Asp Pro Gly Ala Met Arg Ile Leu Pro Leu Ile Ser Pro Ser Glu Pro
                   245                 250                 255
   ggg cac ttt gtt aat ggg ttt gcc gca aac gac gct tct tct act ttg      816
   Gly His Phe Val Asn Gly Phe Ala Ala Asn Asp Ala Ser Ser Thr Leu
               260                 265                 270
   aat ata tgg aaa cgt gta aaa gaa atc ggt tac ccg acc gat gat ccg      864
   Asn Ile Trp Lys Arg Val Lys Glu Ile Gly Tyr Pro Thr Asp Asp Pro
           275                 280                 285
   atc atc atc atg aac tgc cgc gca gac cgt gtc gat cgg aca cag caa      912
   Ile Ile Ile Met Asn Cys Arg Ala Asp Arg Val Asp Arg Thr Gln Gln
       290                 295                 300
   ttc gca aat gac gta ttg cct tat att gaa gca agt gaa ctg atc tta      960
   Phe Ala Asn Asp Val Leu Pro Tyr Ile Glu Ala Ser Glu Leu Ile Leu
   305                 310                 315                 320
   atc ggt gaa aca aca gaa ccg atc gta aaa gcc tac gaa gaa ggc aaa     1008
   Ile Gly Glu Thr Thr Glu Pro Ile Val Lys Ala Tyr Glu Glu Gly Lys
                   325                 330                 335
   att cct gca gac aaa ctg cat gat cta gag tat aag tca aca gat gaa     1056
   Ile Pro Ala Asp Lys Leu His Asp Leu Glu Tyr Lys Ser Thr Asp Glu
               340                 345                 350
   att atg gaa ttg tta aag aaa agt atg cac aac cgt gtc ata tat ggc     1104
   Ile Met Glu Leu Leu Lys Lys Ser Met His Asn Arg Val Ile Tyr Gly
           355                 360                 365
   gtc ggc aat att cat ggt gcc gca gag cct tta att gaa aaa atc cac      1152
   Val Gly Asn Ile His Gly Ala Ala Glu Pro Leu Ile Glu Lys Ile His
       370                 375                 380
   gaa tac aag gta aag cag ctc gta agc tag                              1182
   Glu Tyr Lys Val Lys Gln Leu Val Ser
   385                 390
<210> 2
   <211> 393
   <212> PRT
   <213> Bacillus subtilis
<400> 2
   Met Trp Leu Leu Ile Ile Ala Cys Ala Val Ile Leu Val Ile Gly Ile
   1               5                   10                  15
   Leu Glu Lys Arg Arg His Gln Lys Asn Ile Asp Ala Leu Pro Val Arg
               20                  25                  30
   Val Asn Ile Asn Gly Ile Arg Gly Lys Ser Thr Val Thr Arg Leu Thr
           35                  40                  45
   Thr Gly Ile Leu Ile Glu Ala Gly Tyr Lys Thr Val Gly Lys Thr Thr
       50                  55                  60
   Gly Thr Asp Ala Arg Met Ile Tyr Trp Asp Thr Pro Glu Glu Lys Pro
   65                  70                  75                  80
   Ile Lys Arg Lys Pro Gln Gly Pro Asn Ile Gly Glu Gln Lys Glu Val
                   85                  90                  95
   Met Arg Glu Thr Val Glu Arg Gly Ala Asn Ala Ile Val Ser Glu Cys
               100                 105                 110
   Met Ala Val Asn Pro Asp Tyr Gln Ile Ile Phe Gln Glu Glu Leu Leu
           115                 120                 125
   Gln Ala Asn Ile Gly Val Ile Val Asn Val Leu Glu Asp His Met Asp
       130                 135                 140
   Val Met Gly Pro Thr Leu Asp Glu Ile Ala Glu Ala Phe Thr Ala Thr
   145                 150                 155                 160
   Ile Pro Tyr Asn Gly His Leu Val Ile Thr Asp Ser Glu Tyr Thr Glu
                   165                 170                 175
   Phe Phe Lys Gln Lys Ala Lys Glu Arg Asn Thr Lys Val Ile Ile Ala
             180                 185                 190
   Asp Asn Ser Lys Ile Thr Asp Glu Tyr Leu Arg Lys Phe Glu Tyr Met
           195                 200                 205
   Val Phe Pro Asp Asn Ala Ser Leu Ala Leu Gly Val Ala Gln Ala Leu
       210                 215                 220
   Gly Ile Asp Glu Glu Thr Ala Phe Lys Gly Met Leu Asn Ala Pro Pro
   225                 230                 235                 240
   Asp Pro Gly Ala Met Arg Ile Leu Pro Leu Ile Ser Pro Ser Glu Pro
                   245                 250                 255
   Gly His Phe Val Asn Gly Phe Ala Ala Asn Asp Ala Ser Ser Thr Leu
               260                 265                 270
   Asn Ile Trp Lys Arg Val Lys Glu Ile Gly Tyr Pro Thr Asp Asp Pro
           275                 280                 285
   Ile Ile Ile Met Asn Cys Arg Ala Asp Arg Val Asp Arg Thr Gln Gln
       290                 295                 300
   Phe Ala Asn Asp Val Leu Pro Tyr Ile Glu Ala Ser Glu Leu Ile Leu
   305                 310                 315                 320
   Ile Gly Glu Thr Thr Glu Pro Ile Val Lys Ala Tyr Glu Glu Gly Lys
                   325                 330                 335
   Ile Pro Ala Asp Lys Leu His Asp Leu Glu Tyr Lys Ser Thr Asp Glu
               340                 345                 350
   Ile Met Glu Leu Leu Lys Lys Ser Met His Asn Arg Val Ile Tyr Gly
           355                 360                 365
   Val Gly Asn Ile His Gly Ala Ala Glu Pro Leu Ile Glu Lys Ile His
       370                 375                 380
   Glu Tyr Lys Val Lys Gln Leu Val Ser
   385                 390
<210> 3
   <211> 450
   <212> DNA
   <213> Bacillus subtilis
<220>
   <221> CDS
   <222> (1)..(450)
<400> 3
   atg ttc gga tca gat tta tac atc gca cta att tta ggt gta cta ctc       48
   Met Phe Gly Ser Asp Leu Tyr Ile Ala Leu Ile Leu Gly Val Leu Leu
   1               5                   10                  15
   agt tta att ttt gcg gaa aaa aca ggg atc gtg ccg gca gga ctt gtt       96
   Ser Leu Ile Phe Ala Glu Lys Thr Gly Ile Val Pro Ala Gly Leu Val
               20                  25                  30
   gta ccg gga tat tta gga ctt gtg ttt aat cag ccg gtc ttt att tta      144
   Val Pro Gly Tyr Leu Gly Leu Val Phe Asn Gln Pro Val Phe Ile Leu
           35                  40                  45
   ctt gtt ttg cta gtg agc ttg ctc acg tat gtc att gtg aaa tac ggt      192
   Leu Val Leu Leu Val Ser Leu Leu Thr Tyr Val Ile Val Lys Tyr Gly
       50                  55                  60
   tta tcc aaa ttt atg att ttg tac gga cgc aga aaa ttc gct gcc atg      240
   Leu Ser Lys Phe Met Ile Leu Tyr Gly Arg Arg Lys Phe Ala Ala Met
   65                  70                  75                  80
   ctg ata aca ggg atc gtc cta aaa atc gcg ttt gat ttt cta tac ccg      288
   Leu Ile Thr Gly Ile Val Leu Lys Ile Ala Phe Asp Phe Leu Tyr Pro
                   85                  90                  95
   att gta cca ttt gaa atc gca gaa ttt cga gga atc ggc atc atc gtg      336
   Ile Val Pro Phe Glu Ile Ala Glu Phe Arg Gly Ile Gly Ile Ile Val
               100                 105                 110
   cca ggt tta att gcc aat acc att cag aaa caa ggt tta acc att acg      384
   Pro Gly Leu Ile Ala Asn Thr Ile Gln Lys Gln Gly Leu Thr Ile Thr
           115                 120                 125
   ttc gga agc acg ctg cta ttg agc gga gcg acc ttt gct atc atg ttt      432
   Phe Gly Ser Thr Leu Leu Leu Ser Gly Ala Thr Phe Ala Ile Met Phe
       130                 135                 140
   gtt tac tac tta att taa                                              450
   Val Tyr Tyr Leu Ile
   145
<210> 4
   <211> 149
   <212> PRT
   <213> Bacillus subtilis
<400> 4
   Met Phe Gly Ser Asp Leu Tyr Ile Ala Leu Ile Leu Gly Val Leu Leu
   1               5                   10                  15
   Ser Leu Ile Phe Ala Glu Lys Thr Gly Ile Val Pro Ala Gly Leu Val
               20                  25                  30
   Val Pro Gly Tyr Leu Gly Leu Val Phe Asn Gln Pro Val Phe Ile Leu
           35                  40                  45
   Leu Val Leu Leu Val Ser Leu Leu Thr Tyr Val Ile Val Lys Tyr Gly
       50                  55                  60
   Leu Ser Lys Phe Met Ile Leu Tyr Gly Arg Arg Lys Phe Ala Ala Met
   65                  70                  75                  80
   Leu Ile Thr Gly Ile Val Leu Lys Ile Ala Phe Asp Phe Leu Tyr Pro
                   85                  90                  95
   Ile Val Pro Phe Glu Ile Ala Glu Phe Arg Gly Ile Gly Ile Ile Val
               100                 105                 110
   Pro Gly Leu Ile Ala Asn Thr Ile Gln Lys Gln Gly Leu Thr Ile Thr
           115                 120                 125
   Phe Gly Ser Thr Leu Leu Leu Ser Gly Ala Thr Phe Ala Ile Met Phe
       130                 135                 140
   Val Tyr Tyr Leu Ile
   145
<210> 5
   <211> 1143
   <212> DNA
   <213> Bacillus subtilis
<220>
   <221> CDS
   <222> (1)..(1143)
<400> 5
   atg aaa aaa gaa ctg agc ttt cat gaa aag ctg cta aag ctg aca aaa        48
   Met Lys Lys Glu Leu Ser Phe His Glu Lys Leu Leu Lys Leu Thr Lys
   1               5                   10                  15
   cag caa aaa aag aaa acc aat aag cac gta ttt att gcc att ccg atc        96
   Gln Gln Lys Lys Lys Thr Asn Lys His Val Phe Ile Ala Ile Pro Ile
               20                  25                  30
   gtt ttt gtc ctt atg ttc gct ttc atg tgg gcg gga aaa gcg gaa acg       144
   Val Phe Val Leu Met Phe Ala Phe Met Trp Ala Gly Lys Ala Glu Thr
           35                  40                  45
   ccg aag gtc aaa acg tat tct gac gac gta ctc tca gcc tca ttt gta      192
   Pro Lys Val Lys Thr Tyr Ser Asp Asp Val Leu Ser Ala Ser Phe Val
       50                  55                  60
   ggc gat att atg atg gga cgc tat gtt gaa aaa gta acg gag caa aaa      240
   Gly Asp Ile Met Met Gly Arg Tyr Val Glu Lys Val Thr Glu Gln Lys
   65                  70                  75                  80
   ggg gca gac agt att ttt caa tat gtt gaa ccg atc ttt aga gcc tcg      288
   Gly Ala Asp Ser Ile Phe Gln Tyr Val Glu Pro Ile Phe Arg Ala Ser
                   85                  90                  95
   gat tat gta gca gga aac ttt gaa aac ccg gta acc tat caa aag aat      336
   Asp Tyr Val Ala Gly Asn Phe Glu Asn Pro Val Thr Tyr Gln Lys Asn
               100                 105                 110
   tat aaa caa gca gat aaa gag att cat ctg cag acg aat aag gaa tca      384
   Tyr Lys Gln Ala Asp Lys Glu Ile His Leu Gln Thr Asn Lys Glu Ser
           115                 120                 125
   gtg aaa gtc ttg aag gat atg aat ttc acg gtt ctc aac agc gcc aac      432
   Val Lys Val Leu Lys Asp Met Asn Phe Thr Val Leu Asn Ser Ala Asn
       130                 135                 140
   aac cac gca atg gat tac ggc gtt cag ggc atg aaa gat acg ctt gga      480
   Asn His Ala Met Asp Tyr Gly Val Gln Gly Met Lys Asp Thr Leu Gly
   145                 150                 155                 160
   gaa ttt gcg aag caa aat ctt gat atc gtt gga gcg gga tac agc tta      528
   Glu Phe Ala Lys Gln Asn Leu Asp Ile Val Gly Ala Gly Tyr Ser Leu
                   165                 170                 175
   agt gat gcg aaa aag aaa att tcg tac cag aaa gtc aac ggg gta acg      576
   Ser Asp Ala Lys Lys Lys Ile Ser Tyr Gln Lys Val Asn Gly Val Thr
               180                 185                 190
   att gcg acg ctt ggc ttt acc gat gtg tcc ggg aaa ggt ttc gcg gct      624
   Ile Ala Thr Leu Gly Phe Thr Asp Val Ser Gly Lys Gly Phe Ala Ala
           195                 200                 205
   aaa aag aat acg ccg ggc gtg ctg ccc gca gat cct gaa atc ttc atc      672
   Lys Lys Asn Thr Pro Gly Val Leu Pro Ala Asp Pro Glu Ile Phe Ile
       210                 215                 220
   cct atg att tca gaa gcg aaa aaa cat gcg gac att gtt gtt gtg cag      720
   Pro Met Ile Ser Glu Ala Lys Lys His Ala Asp Ile Val Val Val Gln
   225                 230                 235                 240
   tca cac tgg gga caa gag tat gac aat gat cca aat gac cgc cag cgc      768
   Ser His Trp Gly Gln Glu Tyr Asp Asn Asp Pro Asn Asp Arg Gln Arg
                   245                 250                 255
   cag ctt gca aga gcc atg tct gat gcg gga gct gac atc atc gtc ggc      816
   Gln Leu Ala Arg Ala Met Ser Asp Ala Gly Ala Asp Ile Ile Val Gly
               260                 265                 270
   cat cac ccg cac gtc tta gaa ccg att gaa gta tat aac gga acc gtc      864
   His His Pro His Val Leu Glu Pro Ile Glu Val Tyr Asn Gly Thr Val
           275                 280                 285
   att ttc tac agc ctc ggc aac ttt gtc ttt gac caa ggc tgg acg aga      912
   Ile Phe Tyr Ser Leu Gly Asn Phe Val Phe Asp Gln Gly Trp Thr Arg
       290                 295                 300
   aca aga gac agt gca ctg gtt cag tat cac ctg aag aaa aat gga aca      960
   Thr Arg Asp Ser Ala Leu Val Gln Tyr His Leu Lys Lys Asn Gly Thr
   305                 310                 315                 320
   gga cgc ttt gaa gtg aca ccg atc gat atc cat gaa gcg aca cct gcg     1008
   Gly Arg Phe Glu Val Thr Pro Ile Asp Ile His Glu Ala Thr Pro Ala
                   325                 330                 335
   cct gtg aaa aaa gac agc ctt aaa cag aaa acc att att cgc gaa ctg     1056
   Pro Val Lys Lys Asp Ser Leu Lys Gln Lys Thr Ile Ile Arg Glu Leu
               340                 345                 350
   acg aaa gac tct aat ttc gct tgg aaa gta gaa gac gga aaa ctg acg     1104
   Thr Lys Asp Ser Asn Phe Ala Trp Lys Val Glu Asp Gly Lys Leu Thr
           355                 360                 365
   ttt gat att gat cat agt gac aaa cta aaa tct aaa taa                 1143
   Phe Asp Ile Asp His Ser Asp Lys Leu Lys Ser Lys
       370                 375                 380
<210> 6
   <211> 380
   <212> PRT
   <213> Bacillus subtilis
<400> 6
   Met Lys Lys Glu Leu Ser Phe His Glu Lys Leu Leu Lys Leu Thr Lys
   1               5                   10                  15
   Gln Gln Lys Lys Lys Thr Asn Lys His Val Phe Ile Ala Ile Pro Ile
               20                  25                  30
   Val Phe Val Leu Met Phe Ala Phe Met Trp Ala Gly Lys Ala Glu Thr
           35                  40                  45
   Pro Lys Val Lys Thr Tyr Ser Asp Asp Val Leu Ser Ala Ser Phe Val
       50                  55                  60
   Gly Asp Ile Met Met Gly Arg Tyr Val Glu Lys Val Thr Glu Gln Lys
   65                  70                  75                  80
   Gly Ala Asp Ser Ile Phe Gln Tyr Val Glu Pro Ile Phe Arg Ala Ser
                   85                  90                  95
   Asp Tyr Val Ala Gly Asn Phe Glu Asn Pro Val Thr Tyr Gln Lys Asn
               100                 105                 110
   Tyr Lys Gln Ala Asp Lys Glu Ile His Leu Gln Thr Asn Lys Glu Ser
   115 120 125
   Val Lys Val Leu Lys Asp Met Asn Phe Thr Val Leu Asn Ser Ala Asn
   130 135 140
   Asn His Ala Met Asp Tyr Gly Val Gln Gly Met Lys Asp Thr Leu Gly
   145 150 155 160
   Glu Phe Ala Lys Gln Asn Leu Asp Ile Val Gly Ala Gly Tyr Ser Leu
   165 170 175
   Ser Asp Ala Lys Lys Lys Ile Ser Tyr Gln Lys Val Asn Gly Val Thr
   180 185 190
   Ile Ala Thr Leu Gly Phe Thr Asp Val Ser Gly Lys Gly Phe Ala Ala
   195 200 205
   Lys Lys Asn Thr Pro Gly Val Leu Pro Ala Asp Pro Glu Ile Phe Ile
   210 215 220
   Pro Met Ile Ser Glu Ala Lys Lys His Ala Asp Ile Val Val Val Gln
   225 230 235 240
   Ser His Trp Gly Gln Glu Tyr Asp Asn Asp Pro Asn Asp Arg Gln Arg
   245 250 255
   Gln Leu Ala Arg Ala Met Ser Asp Ala Gly Ala Asp Ile Ile Val Gly
   260 265 270
   His His Pro His Val Leu Glu Pro Ile Glu Val Tyr Asn Gly Thr Val
   275 280 285
   Ile Phe Tyr Ser Leu Gly Asn Phe Val Phe Asp Gln Gly Trp Thr Arg
   290 295 300
   Thr Arg Asp Ser Ala Leu Val Gln Tyr His Leu Lys Lys Asn Gly Thr
   305 310 315 320
   Gly Arg Phe Glu Val Thr Pro Ile Asp Ile His Glu Ala Thr Pro Ala
   325 330 335
   Pro Val Lys Lys Asp Ser Leu Lys Gln Lys Thr Ile Ile Arg Glu Leu
   340 345 350
   Thr Lys Asp Ser Asn Phe Ala Trp Lys Val Glu Asp Gly Lys Leu Thr
           355                 360                 365
   Phe Asp Ile Asp His Ser Asp Lys Leu Lys Ser Lys
       370                 375                 380
<210> 7
   <211> 1761
   <212> DNA
   <213> Bacillus subtilis
<220>
   <221> CDS
   <222> (1)..(1761)
<400> 7
<210> 8
   <211> 587
   <212> PRT
   <213> Bacillus subtilis
<400> 8
   Met Lys Arg Thr Trp Asn Val Cys Leu Thr Ala Leu Leu Ser Val Leu
   1               5                   10                  15
   Leu Val Ala Gly Ser Val Pro Phe His Ala Glu Ala Lys Lys Pro Pro
   20 25 30
   Lys Ser Tyr Asp Glu Tyr Lys Gln Val Asp Val Gly Lys Asp Gly Met
   35 40 45
   Val Ala Thr Ala His Pro Leu Ala Ser Glu Ile Gly Ala Asp Val Leu
   50 55 60
   Lys Lys Gly Gly Asn Ala Ile Asp Ala Ala Val Ala Ile Gln Phe Ala
   65 70 75 80
   Leu Asn Val Thr Glu Pro Met Met Ser Gly Ile Gly Gly Gly Gly Phe
   85 90 95
   Met Met Val Tyr Asp Gly Lys Thr Lys Asp Thr Thr Ile Ile Asp Ser
   100 105 110
   Arg Glu Arg Ala Pro Ala Gly Ala Thr Pro Asp Met Phe Leu Asp Glu
   115 120 125
   Asn Gly Lys Ala Ile Pro Phe Ser Glu Arg Val Thr Lys Gly Thr Ala
   130 135 140
   Val Gly Val Pro Gly Thr Leu Lys Gly Leu Glu Glu Ala Leu Asp Lys
   145 150 155 160
   Trp Gly Thr Arg Ser Met Lys Gln Leu Ile Thr Pro Ser Ile Lys Leu
   165 170 175
   Ala Glu Lys Gly Phe Pro Ile Asp Ser Val Leu Ala Glu Ala Ile Ser
   180 185 190
   Asp Tyr Gln Glu Lys Leu Ser Arg Thr Ala Ala Lys Asp Val Phe Leu
   195 200 205
   Pro Asn Gly Glu Pro Leu Lys Glu Gly Asp Thr Leu Ile Gln Lys Asp
   210 215 220
   Leu Ala Lys Thr Phe Lys Leu Ile Arg Ser Lys Gly Thr Asp Ala Phe
   225 230 235 240
   Tyr Lys Gly Lys Phe Ala Lys Thr Leu Ser Asp Thr Val Gln Asp Phe
   245 250 255
   Gly Gly Ser Met Thr Glu Lys Asp Leu Glu Asn Tyr Asp Ile Thr Ile
   260 265 270
   Asp Glu Pro Ile Trp Gly Asp Tyr Gln Gly Tyr Gln Ile Ala Thr Thr
   275 280 285
   Pro Pro Pro Ser Ser Gly Gly Ile Phe Leu Leu Gln Met Leu Lys Ile
   290 295 300
   Leu Asp His Phe Asn Leu Ser Gln Tyr Asp Val Arg Ser Trp Glu Lys
   305 310 315 320
   Tyr Gln Leu Leu Ala Glu Thr Met His Leu Ser Tyr Ala Asp Arg Ala
   325 330 335
   Ser Tyr Ala Gly Asp Pro Glu Phe Val Asn Val Pro Leu Lys Gly Leu
   340 345 350
   Leu His Pro Asp Tyr Ile Lys Glu Arg Gln Gln Leu Ile Asn Leu Asp
   355 360 365
   Gln Val Asn Lys Lys Pro Lys Ala Gly Asp Pro Trp Lys Tyr Gln Glu
   370 375 380
   Gly Ser Ala Asn Tyr Lys Gln Val Glu Gln Pro Lys Asp Lys Val Glu
   385 390 395 400
   Gly Gln Thr Thr His Phe Thr Val Ala Asp Arg Trp Gly Asn Val Val
   405 410 415
   Ser Tyr Thr Thr Thr Ile Glu Gln Leu Phe Gly Thr Gly Ile Met Val
   420 425 430
   Pro Asp Tyr Gly Val Ile Leu Asn Asn Glu Leu Thr Asp Phe Asp Ala
   435 440 445
   Ile Pro Gly Gly Ala Asn Glu Val Gln Pro Asn Lys Arg Pro Leu Ser
   450 455 460
   Ser Met Thr Pro Thr Ile Leu Phe Lys Asp Asp Lys Pro Val Leu Thr
   465 470 475 480
   Val Gly Ser Pro Gly Gly Ala Thr Ile Ile Ser Ser Val Leu Gln Thr
   485 490 495
   Ile Leu Tyr His Ile Glu Tyr Gly Met Glu Leu Lys Ala Ala Val Glu
   500 505 510
   Glu Pro Arg Ile Tyr Thr Asn Ser Met Ser Ser Tyr Arg Tyr Glu Asp
   515 520 525
   Gly Val Pro Lys Asp Val Leu Ser Lys Leu Asn Gly Met Gly His Lys
   530 535 540
   Phe Gly Thr Ser Pro Val Asp Ile Gly Asn Val Gln Ser Ile Ser Ile
   545 550 555 560
   Asp His Glu Asn Gly Thr Phe Lys Gly Val Ala Asp Ser Ser Arg Asn
   565 570 575
   Gly Ala Ala Ile Gly Ile Asn Leu Lys Arg Lys
   580 585
<210> 9
   <211> 575
   <212> DNA
   <213> Bacillus subtilis
<400> 9
   gatttgccga tgcaacaggc ttatatttag aggaaatttc tttttaaatt gaatacggaa 60
   taaaatcagg taaacaggtc ctgattttat ttttttgagt tttttagaga actgaagatt 120
   gaaataaaag tagaagacaa aggacataag aaaattgcat tagttttaat tatagaaaac 180
   gcctttttat aattatttat acctagaacg aaaatactgt ttcgaaagcg gtttactata 240
   aaaccttata ttccggctct tttttaaaac agggggtaaa aattcactct agtattctaa 300
   tttcaacatg ctataataaa tttgtaagac gcaatatgca tctctttttt tacgatatat 360
   gtaagcggtt aaccttgtgc tatatgccga tttaggaagg ggggtagatt gagtcaagta 420
   gtaataatat agataactta taagttgttg agaagcagga gagcatctgg gttactcaca 480
   agttttttta aaactttaac gaaagcactt tcggtaatgc ttatgaattt agctatttga 540
   ttcaattact ttaaaaatat ttaggaggta atatg 575
<210> 10
   <211> 567
   <212> DNA
   <213> Bacillus subtilis
<400> 10
   caaaagcagt ccacacaaaa catgccccag cggcaatcgg gccttattca caagggatta 60
   tcgtcaacaa tatgttttac agctcaggcc aaatcccttt gactccttca ggcgaaatgg 120
   tgaatggcga tattaaggag cagactcatc aagtattcag caatttaaag gcggttctgg 180
   aagaagcggg tgcttctttt gaaacagttg taaaagcaac tgtatttatc gcggatatgg 240
   aacagtttgc ggaagtaaac gaagtgtacg gacaatattt tgacactcac aaaccggcga 300
   gatcttgtgt tgaagtcgcg agactcccga aggatgcgtt agtcgagatc gaagttattg 360
   cactggtgaa ataataagaa aagtgattct gggagagccg ggatcacttt tttatttacc 420
   ttatgcccga aatgaaagct ttatgaccta attgtgtaac tatatcctat tttttcaaaa 480
   aatattttaa aaacgagcag gatttcagaa aaaatcgtgg aattgataca ctaatgcttt 540
   tatataggga aaaggtggtg aactact 567
<210> 11
   <211> 500
   <212> DNA
   <213> Bacillus subtilis
<400> 11
   tatttctcat cggtacgaca aactatcccg aagagatcga tccaggtttg atgaatcgtg 60
   caggacgatt tgaccgtgcc tatgaaatcg ggcttccgga tgaagagctg cggctggaat 120
   atatgaaaat gagaggcttt ggcatctttt tgagtgaagg agaaataaaa aacgccgcaa 180
   aacttacaga aggcttttcc tttgcacagc tgggagaatt atatgtatct tcagcccttc 240
   aatggcacca agaagggaat caccatattg aaaccatggt gaaagacatg acaggagagc 300
   aaagaaaaag ccagcgggga agctggatgg aaagaaacaa agtcggtttt cactaaaaga 360
   aagcacgggt gtttgaaaaa cccgtgcttt tttgttgcgg ttagccgaaa ttcgacaatt 420
   gcggttattt tgcgttcttc tttttcttgt aaatatgata aaatatgaca tatctcgggt 480
   aattcaaaag gggggattaa 500
<210> 12
   <211> 40
   <212> DNA
   <213> Artificial
<220>
   <221> source
   <223> /note="Description of artificial sequence: Oligonucleotide as PCR forward primer for amplifying pgsBCA region"
<400> 12
   atttaggagg taatatgatg tggttactca ttatagcctg 40
<210> 13
   <211> 31
   <212> DNA
   <213> Artificial
<220>
   <221> source
   <223> /note="Description of artificial sequence: Oligonucleotide as PCR reverse primer for amplifying pgsBCA region"
<400> 13
   cgaagcttag atggctttga caaatttcat c 31
<210> 14
   <211> 28
   <212> DNA
   <213> Artificial
<220>
   <221> source
   <223> /note="Description of artificial sequence: Oligonucleotide as PCR forward primer for amplifying pgsBC region"
<400> 14
   cccaagcttg accttcggcg tttccgct 28
<210> 15
   <211> 30
   <212> DNA
   <213> Artificial
<220>
   <221> source
   <223> /note="Description of artificial sequence: Oligonucleotide as PCR reverse primer for amplifying pgsBC region"
<400> 15
   cccaagcttg gcagcgaatt ttctgcgtcc 30
<210> 16
   <211> 44
   <212> DNA
   <213> Artificial
<220>
   <221> source
   <223> /note="Description of artificial sequence: Oligonucleotide as PCR forward primer for amplifying a promoter region of S237 cellulase gene"
<400> 16
   caactaaagc acccattagg gatccaacag gcttatattt agag 44
<210> 17
   <211> 32
   <212> DNA
   <213> Artificial
<220>
   <221> source
   <223> /note="Description of artificial sequence: Oligonucleotide as PCR reverse primer for amplifying a promoter region of S237 cellulase gene"
<400> 17
   catcatatta cctcctaaat atttttaaag ta 32
<210> 18
   <211> 20
   <212> DNA
   <213> Artificial
<220>
   <221> source
   <223> /note="Description of artificial sequence: Oligonucleotide as PCR forward primer for amplifying an upstream region of ggt gene"
<400> 18
   tccttcatgt ctttcgtata 20
<210> 19
   <211> 38
   <212> DNA
   <213> Artificial
<220>
   <221> source
   <223> /note="Description of artificial sequence: Oligonucleotide as PCR reverse primer for amplifying an upstream region of ggt gene"
<400> 19
   ctaatgggtg ctttagttgg ttctccctcc tatatgaa 38
<210> 20
   <211> 39
   <212> DNA
   <213> Artificial
<220>
   <221> source
   <223> /note="Description of artificial sequence: Oligonucleotide as PCR forward primer for amplifying a downstream region of ggt gene"
<400> 20
   ctgccccgtt agttgaagat aaaaaactgt actcgcttc 39
<210> 21
   <211> 21
   <212> DNA
   <213> Artificial
<220>
   <221> source
   <223> /note="Description of artificial sequence: Oligonucleotide as PCR reverse primer for amplifying a downstream region of ggt gene"
<400> 21
   tagccaatat cacttttcat c 21
<210> 22
   <211> 30
   <212> DNA
   <213> Artificial
<220>
   <221> source
   <223> /note="Description of artificial sequence: Oligonucleotide as PCR forward primer for amplifying a region including chloramphenicol-resistant gene"
<400> 22
   caactaaagc acccattagt tcaacaaacg 30
<210> 23
   <211> 27
   <212> DNA
   <213> Artificial
<220>
   <221> source
   <223> /note="Description of artificial sequence: Oligonucleotide as PCR reverse primer for amplifying a region including chloramphenicol-resistant gene"
<400> 23
   cttcaactaa cggggcaggt tagtgac 27
<210> 24
   <211> 20
   <212> DNA
   <213> Artificial
<220>
   <221> source
   <223> /note="Description of artificial sequence: Oligonucleotide as PCR forward primer for amplifying spoVG-UP fragment"
<400> 24
   atgaagtttc gtcgcagcgg 20
<210> 25
   <211> 43
   <212> DNA
   <213> Artificial
<220>
   <221> source
   <223> /note="Description of artificial sequence: Oligonucleotide as PCR reverse primer for amplifying spoVG-UP fragment"
<400> 25
   ctaatgggtg ctttagttgt catgattctg tctctccatt ctt 43
<210> 26
   <211> 42
   <212> DNA
   <213> Artificial
<220>
   <221> source
   <223> /note="Description of artificial sequence: Oligonucleotide as PCR forward primer for amplifying spoVG-DW fragment"
<400> 26
   ctgccccgtt agttgaagca aaagcagtcc acacaaaaca tg 42
<210> 27
   <211> 28
   <212> DNA
   <213> Artificial
<220>
   <221> source
   <223> /note="Description of artificial sequence: Oligonucleotide as PCR reverse primer for amplifying spoVG-DW fragment"
<400> 27
   catagtagtt caccaccttt tccctata 28
<210> 28
   <211> 42
   <212> DNA
   <213> Artificial
<220>
   <221> source
   <223> /note="Description of artificial sequence: Oligonucleotide as PCR forward primer for amplifying comp_Cm fragment"
<400> 28
   caactaaagc acccattagg ttagtgacat tagaaaaccg ac 42
<210> 29
   <211> 45
   <212> DNA
   <213> Artificial
<220>
   <221> source
   <223> /note="Description of artificial sequence: Oligonucleotide as PCR reverse primer for amplifying comp_Cm fragment"
<400> 29
   cttcaactaa cggggcagtt caacaaacga aaattggata aagtg 45
<210> 30
   <211> 48
   <212> DNA
   <213> Artificial
<220>
   <221> source
   <223> /note="Description of artificial sequence: Oligonucleotide as PCR forward primer for amplifying pgsB1 fragment"
<400> 30
   tatagggaaa aggtggtgaa ctactatgtg gttactcatt atagcctg 48
<210> 31
   <211> 24
   <212> DNA
   <213> Artificial
<220>
   <221> source
   <223> /note="Description of artificial sequence: Oligonucleotide as PCR reverse primer for amplifying pgsB1 fragment"
<400> 31
   gtctgcattt ccccctagct tacg 24
<210> 32
   <211> 37
   <212> DNA
   <213> Artificial
<220>
   <221> source
   <223> /note="Description of artificial sequence: Oligonucleotide as PCR forward primer for amplifying pgsB-UP fragment"
<400> 32
   ctgccccgtt agttgaagtg cttttcgaca tctcctt 37
<210> 33
   <211> 19
   <212> DNA
   <213> Artificial
<220>
   <221> source
   <223> /note="Description of artificial sequence: Oligonucleotide as PCR reverse primer for amplifying pgsB-UP fragment"
<400> 33
   aagggtttgt gatatccgg 19
<210> 34
   <211> 42
   <212> DNA
   <213> Artificial
<220> source
   <223> /note="Description of artificial sequence: Oligonucleotide as PCR reverse primer for amplifying P_spoVG/pgsBC fragment"
<400> 34
   ctgccccggg atccgaagca aaagcagtcc acacaaaaca tg 42
<210> 35
   <211> 20
   <212> DNA
   <213> Artificial
<220>
   <221> source
   <223> /note="Description of artificial sequence: Oligonucleotide as PCR forward primer for amplifying rapA-UP fragment"
<400> 35
   tgaaaagatg cgtgcatttc 20
<210> 36
   <211> 41
   <212> DNA
   <213> Artificial
<220>
   <221> source
   <223> /note="Description of artificial sequence: Oligonucleotide as PCR reverse primer for amplifying rapA-UP fragment"
<400> 36
   ctaatgggtg ctttagttga atagcccctc ttttgatgtc g 41
<210> 37
   <211> 46
   <212> DNA
   <213> Artificial
<220>
   <221> source
   <223> /note="Description of artificial sequence: Oligonucleotide as PCR forward primer for amplifying rapA-DW fragment"
<400> 37
   ctgccccgtt agttgaagta tttctcatcg gtacgacaaa ctatcc 46
<210> 38
   <211> 23
   <212> DNA
   <213> Artificial
<220>
   <221> source
   <223> /note="Description of artificial sequence: Oligonucleotide as PCR reverse primer for amplifying rapA-DW fragment"
<400> 38
   catttaatcc ccccttttga att 23
<210> 39
   <211> 43
   <212> DNA
   <213> Artificial
<220>
   <221> source
   <223> /note="Description of artificial sequence: Oligonucleotide as PCR forward primer for amplifying pgsB2 fragment"
<400> 39
   aattcaaaag gggggattaa atgtggttac tcattatagc ctg 43
<210> 40
   <211> 46
   <212> DNA
   <213> Artificial
<220>
   <221> source
   <223> /note="Description of artificial sequence: Oligonucleotide as PCR forward primer for amplifying P_rapA/pgsBC fragment"
<400> 40
   ctgccccggg atccgaagta tttctcatcg gtacgacaaa ctatcc 46
<210> 41
   <211> 21
   <212> DNA
   <213> Artificial
<220>
   <221> source
   <223> /note="Description of artificial sequence: Oligonucleotide as PCR forward primer for amplifying tet-P_B fragment"
<400> 41
   catattgttg tataagtgat g 21
<210> 42
   <211> 41
   <212> DNA
   <213> Artificial
<220>
   <221> source
   <223> /note="Description of artificial sequence: Oligonucleotide as PCR forward primer for amplifying pgsB-DW1 fragment"
<400> 42
   catcacttat acaacaatat gtgcttttcg acatctcctt c 41
<210> 43
   <211> 41
   <212> DNA
   <213> Artificial
<220>
   <221> source
   <223> /note="Description of artificial sequence: Oligonucleotide as PCR forward primer for amplifying Cm-PS237 fragment"
<400> 43
   taaatataag cctgttggat cccaactaaa gcacccatta g 41
<210> 44
   <211> 27
   <212> DNA
   <213> Artificial
<220>
   <221> source
   <223> /note="Description of artificial sequence: Oligonucleotide as PCR forward primer for amplifying a fragment of pgsBC gene"
<400> 44
   cccaagcttc ttcaactaac ggggcag 27
<210> 45
   <211> 17
   <212> DNA
   <213> Artificial
<220>
   <221> source
   <223> /note="Description of artificial sequence: Oligonucleotide as PCR forward primer for amplifying BCA-UP fragment"
<400> 45
   caagccccga gcaatca 17
<210> 46
   <211> 38
   <212> DNA
   <213> Artificial
<220>
   <221> source
   <223> /note="Description of artificial sequence: Oligonucleotide as PCR reverse primer for amplifying BCA-UP fragment"
<400> 46
   ctaatgggtg ctttagttgc gaaagactct aatttcgc 38
<210> 47
   <211> 1179
   <212> DNA
   <213> Bacillus subtilis KSM-366
<220>
   <221> CDS
   <222> (1)..(1179)
<400> 47
   atg tgg tta ctc att ata gcc tgt gct gtc ata ctg gtc atc gga ata       48
   Met Trp Leu Leu Ile Ile Ala Cys Ala Val Ile Leu Val Ile Gly Ile
   1               5                   10                  15
   tta gaa aaa cga cga cat cag aaa aac att gat gcc ctc cct gtt cgg       96
   Leu Glu Lys Arg Arg His Gln Lys Asn Ile Asp Ala Leu Pro Val Arg
               20                  25                  30
   gtg aat att aac ggc atc cgc gga aaa tcg act gtg aca agg ctg aca      144
   Val Asn Ile Asn Gly Ile Arg Gly Lys Ser Thr Val Thr Arg Leu Thr
           35                  40                  45
   acc gga ata tta ata gaa gcc ggt tac aag act gtt gga aaa aca aca      192
   Thr Gly Ile Leu Ile Glu Ala Gly Tyr Lys Thr Val Gly Lys Thr Thr
       50                  55                  60
   gga aca gat gca aga atg att tac tgg gac aca ccg gag gaa aag ccg      240
   Gly Thr Asp Ala Arg Met Ile Tyr Trp Asp Thr Pro Glu Glu Lys Pro
   65                  70                  75                  80
   att aaa cgg aaa cct cag ggg ccg aat atc gga gag caa aaa gaa gtc      288
   Ile Lys Arg Lys Pro Gln Gly Pro Asn Ile Gly Glu Gln Lys Glu Val
                   85                  90                  95
   atg aga gaa aca gta gaa aga ggg gct aac gcg att gtc agt gaa tgc      336
   Met Arg Glu Thr Val Glu Arg Gly Ala Asn Ala Ile Val Ser Glu Cys
               100                 105                 110
   atg gct gtt aac cca gat tat caa atc atc ttt cag gaa gaa ctt ctg      384
   Met Ala Val Asn Pro Asp Tyr Gln Ile Ile Phe Gln Glu Glu Leu Leu
           115                 120                 125
   cag gcc aat atc ggc gtc att gtg aat gtt tta gaa gac cat atg gat      432
   Gln Ala Asn Ile Gly Val Ile Val Asn Val Leu Glu Asp His Met Asp
       130                 135                 140
   gtc atg ggg ccg acg ctt gat gaa att gca gaa gcg ttt acc gct aca      480
   Val Met Gly Pro Thr Leu Asp Glu Ile Ala Glu Ala Phe Thr Ala Thr
   145                 150                 155                 160
   att cct tat aat ggc cat ctt gtc att aca gat agt gaa tat acc gag      528
   Ile Pro Tyr Asn Gly His Leu Val Ile Thr Asp Ser Glu Tyr Thr Glu
                   165                 170                 175
   ttc ttt aaa caa aaa gca aaa gaa cga aac aca aaa gtc atc att gct      576
   Phe Phe Lys Gln Lys Ala Lys Glu Arg Asn Thr Lys Val Ile Ile Ala
               180                 185                 190
   gat aac tca aaa att aca gat gag tat tta cgt aaa ttt gaa tac atg      624
   Asp Asn Ser Lys Ile Thr Asp Glu Tyr Leu Arg Lys Phe Glu Tyr Met
           195                 200                 205
   gta ttc cct gat aac gct tct ctg gcg ctg ggt gtg gct caa gca ctc      672
   Val Phe Pro Asp Asn Ala Ser Leu Ala Leu Gly Val Ala Gln Ala Leu
       210                 215                 220
   ggc att gac gaa gaa aca gca ttt aag gga atg ctg aat gcg ccg cca        720
   Gly Ile Asp Glu Glu Thr Ala Phe Lys Gly Met Leu Asn Ala Pro Pro
   225                 230                 235                 240
   gat ccg gga gca atg aga att ctt ccg ctg atc agt ccg agc gag cct        768
   Asp Pro Gly Ala Met Arg Ile Leu Pro Leu Ile Ser Pro Ser Glu Pro
                   245                 250                 255
   ggg cac ttt gtt aat ggg ttt gcc gca aac gac gct tct tct act ttg        816
   Gly His Phe Val Asn Gly Phe Ala Ala Asn Asp Ala Ser Ser Thr Leu
               260                 265                 270
   aat ata tgg aaa cgt gta aaa gaa atc ggt tac ccg acc gat gat ccg        864
   Asn Ile Trp Lys Arg Val Lys Glu Ile Gly Tyr Pro Thr Asp Asp Pro
           275                 280                 285
   atc atc atc atg aac tgc cgc gca gac cgt gtc gat cgg aca cag caa        912
   Ile Ile Ile Met Asn Cys Arg Ala Asp Arg Val Asp Arg Thr Gln Gln
       290                 295                 300
   ttc gca aat gac gta ttg cct tat att gaa gca agt gaa ctg atc tta        960
   Phe Ala Asn Asp Val Leu Pro Tyr Ile Glu Ala Ser Glu Leu Ile Leu
   305                 310                 315                 320
   atc ggt gaa aca aca gaa ccg atc gta aaa gcc tac gaa gaa ggc aaa       1008
   Ile Gly Glu Thr Thr Glu Pro Ile Val Lys Ala Tyr Glu Glu Gly Lys
                   325                 330                 335
   att cct gca gac aaa ctg cat gat cta gag tat aag tca aca gat gaa       1056
   Ile Pro Ala Asp Lys Leu His Asp Leu Glu Tyr Lys Ser Thr Asp Glu
               340                 345                 350
   att atg gaa ttg tta aag aaa aga atg cac aac cgt gtc ata tat ggc       1104
   Ile Met Glu Leu Leu Lys Lys Arg Met His Asn Arg Val Ile Tyr Gly
           355                 360                 365
   gtc ggc aat att cat ggt gcc gca gag cct tta att gaa aaa atc cac       1152
   Val Gly Asn Ile His Gly Ala Ala Glu Pro Leu Ile Glu Lys Ile His
       370                 375                 380
   gaa tac aag gtt aag cag ctc gta agc                                   1179
   Glu Tyr Lys Val Lys Gln Leu Val Ser
   385                 390
<210> 48
   <211> 393
   <212> PRT
   <213> Bacillus subtilis KSM-366
<400> 48
   Met Trp Leu Leu Ile Ile Ala Cys Ala Val Ile Leu Val Ile Gly Ile
   1               5                   10                  15
   Leu Glu Lys Arg Arg His Gln Lys Asn Ile Asp Ala Leu Pro Val Arg
               20                  25                  30
   Val Asn Ile Asn Gly Ile Arg Gly Lys Ser Thr Val Thr Arg Leu Thr
           35                  40                  45
   Thr Gly Ile Leu Ile Glu Ala Gly Tyr Lys Thr Val Gly Lys Thr Thr
       50                  55                  60
   Gly Thr Asp Ala Arg Met Ile Tyr Trp Asp Thr Pro Glu Glu Lys Pro
   65                  70                  75                  80
   Ile Lys Arg Lys Pro Gln Gly Pro Asn Ile Gly Glu Gln Lys Glu Val
                   85                  90                  95
   Met Arg Glu Thr Val Glu Arg Gly Ala Asn Ala Ile Val Ser Glu Cys
               100                 105                 110
   Met Ala Val Asn Pro Asp Tyr Gln Ile Ile Phe Gln Glu Glu Leu Leu
           115                 120                 125
   Gln Ala Asn Ile Gly Val Ile Val Asn Val Leu Glu Asp His Met Asp
       130                 135                 140
   Val Met Gly Pro Thr Leu Asp Glu Ile Ala Glu Ala Phe Thr Ala Thr
   145                 150                 155                 160
   Ile Pro Tyr Asn Gly His Leu Val Ile Thr Asp Ser Glu Tyr Thr Glu
                   165                 170                 175
   Phe Phe Lys Gln Lys Ala Lys Glu Arg Asn Thr Lys Val Ile Ile Ala
               180                 185                 190
   Asp Asn Ser Lys Ile Thr Asp Glu Tyr Leu Arg Lys Phe Glu Tyr Met
           195                 200                 205
   Val Phe Pro Asp Asn Ala Ser Leu Ala Leu Gly Val Ala Gln Ala Leu
       210                 215                 220
   Gly Ile Asp Glu Glu Thr Ala Phe Lys Gly Met Leu Asn Ala Pro Pro
   225                 230                 235                 240
   Asp Pro Gly Ala Met Arg Ile Leu Pro Leu Ile Ser Pro Ser Glu Pro
                   245                 250                 255
   Gly His Phe Val Asn Gly Phe Ala Ala Asn Asp Ala Ser Ser Thr Leu
               260                 265                 270
   Asn Ile Trp Lys Arg Val Lys Glu Ile Gly Tyr Pro Thr Asp Asp Pro
           275                 280                 285
   Ile Ile Ile Met Asn Cys Arg Ala Asp Arg Val Asp Arg Thr Gln Gln
       290                 295                 300
   Phe Ala Asn Asp Val Leu Pro Tyr Ile Glu Ala Ser Glu Leu Ile Leu
   305                 310                 315                 320
   Ile Gly Glu Thr Thr Glu Pro Ile Val Lys Ala Tyr Glu Glu Gly Lys
                   325                 330                 335
   Ile Pro Ala Asp Lys Leu His Asp Leu Glu Tyr Lys Ser Thr Asp Glu
               340                 345                 350
   Ile Met Glu Leu Leu Lys Lys Arg Met His Asn Arg Val Ile Tyr Gly
           355                 360                 365
   Val Gly Asn Ile His Gly Ala Ala Glu Pro Leu Ile Glu Lys Ile His
       370                 375                 380
   Glu Tyr Lys Val Lys Gln Leu Val Ser
   385                 390
<210> 49
   <211> 447
   <212> DNA
   <213> Bacillus subtilis KSM-366
<220>
   <221> CDS
   <222> (1)..(447)
<400> 49
   atg ttc gga tca gat tta tac atc gca cta att tta ggt gta cta ctc       48
   Met Phe Gly Ser Asp Leu Tyr Ile Ala Leu Ile Leu Gly Val Leu Leu
   1               5                   10                  15
   agt tta att ttt gcg gaa aaa aca ggg atc gtg ccg gca gga ctt gtt       96
   Ser Leu Ile Phe Ala Glu Lys Thr Gly Ile Val Pro Ala Gly Leu Val
               20                  25                  30
   gta ccg gga tat tta gga ctt gtg ttt aat cag ccg gtc ttt att tta      144
   Val Pro Gly Tyr Leu Gly Leu Val Phe Asn Gln Pro Val Phe Ile Leu
           35                  40                  45
   ctt gtt ttg cta gtg agc ttg ctc act tat gtt atc gtg aaa tac ggt      192
   Leu Val Leu Leu Val Ser Leu Leu Thr Tyr Val Ile Val Lys Tyr Gly
       50                  55                  60
   tta tcc aaa ttt atg att ttg tac gga cgc aga aaa ttc gct gcc atg      240
   Leu Ser Lys Phe Met Ile Leu Tyr Gly Arg Arg Lys Phe Ala Ala Met
   65                  70                  75                  80
   ctg ata aca ggg atc gtc cta aaa atc gcg ttt gat ttt cta tac ccg      288
   Leu Ile Thr Gly Ile Val Leu Lys Ile Ala Phe Asp Phe Leu Tyr Pro
                   85                  90                  95
   att gta cca ttt gaa atc gca gaa ttt cga gga atc ggc atc atc gtg      336
   Ile Val Pro Phe Glu Ile Ala Glu Phe Arg Gly Ile Gly Ile Ile Val
               100                 105                 110
   cca ggt tta att gcc aat acc att cag aaa caa ggt tta acc att acg      384
   Pro Gly Leu Ile Ala Asn Thr Ile Gln Lys Gln Gly Leu Thr Ile Thr
           115                 120                 125
   ttc gga agc acg ctg cta ttg agc gga gcg acc ttt gct atc atg ttt      432
   Phe Gly Ser Thr Leu Leu Leu Ser Gly Ala Thr Phe Ala Ile Met Phe
       130                 135                 140
   gtt tac tac tta att                                                  447
   Val Tyr Tyr Leu Ile
   145
<210> 50
   <211> 149
   <212> PRT
   <213> Bacillus subtilis KSM-366
<400> 50
   Met Phe Gly Ser Asp Leu Tyr Ile Ala Leu Ile Leu Gly Val Leu Leu
   1               5                   10                  15
   Ser Leu Ile Phe Ala Glu Lys Thr Gly Ile Val Pro Ala Gly Leu Val
               20                  25                  30
   Val Pro Gly Tyr Leu Gly Leu Val Phe Asn Gln Pro Val Phe Ile Leu
           35                  40                  45
   Leu Val Leu Leu Val Ser Leu Leu Thr Tyr Val Ile Val Lys Tyr Gly
       50                  55                  60
   Leu Ser Lys Phe Met Ile Leu Tyr Gly Arg Arg Lys Phe Ala Ala Met
   65                  70                  75                  80
   Leu Ile Thr Gly Ile Val Leu Lys Ile Ala Phe Asp Phe Leu Tyr Pro
                   85                  90                  95
   Ile Val Pro Phe Glu Ile Ala Glu Phe Arg Gly Ile Gly Ile Ile Val
               100                 105                 110
   Pro Gly Leu Ile Ala Asn Thr Ile Gln Lys Gln Gly Leu Thr Ile Thr
       115                 120                 125
   Phe Gly Ser Thr Leu Leu Leu Ser Gly Ala Thr Phe Ala Ile Met Phe
       130                 135                 140
   Val Tyr Tyr Leu Ile
   145
<210> 51
   <211> 1140
   <212> DNA
   <213> Bacillus subtilis KSM-366
<220>
   <221> CDS
   <222> (1)..(1140)
<400> 51
   atg aaa aaa gaa ctg agc ttt cat gaa aag ctg cta aag ctg aca aaa       48
   Met Lys Lys Glu Leu Ser Phe His Glu Lys Leu Leu Lys Leu Thr Lys
   1               5                   10                  15
   cag caa aaa aag aaa acc aat aag cac gta ttt att gcc att ccg atc       96
   Gln Gln Lys Lys Lys Thr Asn Lys His Val Phe Ile Ala Ile Pro Ile
               20                  25                  30
   gtt ttt gtc ctt atg ttc gct ttc atg tgg gcg gga aaa gcg gaa acg      144
   Val Phe Val Leu Met Phe Ala Phe Met Trp Ala Gly Lys Ala Glu Thr
           35                  40                  45
   ccg aag gtc aaa acg tat tct gac gac gta ctc tca gcc tca ttt gta      192
   Pro Lys Val Lys Thr Tyr Ser Asp Asp Val Leu Ser Ala Ser Phe Val
       50                  55                  60
   ggc gat att atg atg gga cgc tat gtt gaa aaa gta acg gag caa aaa      240
   Gly Asp Ile Met Met Gly Arg Tyr Val Glu Lys Val Thr Glu Gln Lys
   65                  70                  75                  80
   ggg gca gac agt att ttt caa tat gtt gaa ccg atc ttt aga gcc tcg      288
   Gly Ala Asp Ser Ile Phe Gln Tyr Val Glu Pro Ile Phe Arg Ala Ser
                   85                  90                  95
   gat tat gta gca gga aac ttt gaa aac ccg gta acc tat caa aag aat      336
   Asp Tyr Val Ala Gly Asn Phe Glu Asn Pro Val Thr Tyr Gln Lys Asn
               100                 105                 110
   tat aaa caa gca gat aaa gag att cat ctg cag acg aat aag gaa tca      384
   Tyr Lys Gln Ala Asp Lys Glu Ile His Leu Gln Thr Asn Lys Glu Ser
           115                 120                 125
   gtg aaa gtc ttg aag gat atg aat ttc acg gtt ctc aac agc gca aac      432
   Val Lys Val Leu Lys Asp Met Asn Phe Thr Val Leu Asn Ser Ala Asn
       130                 135                 140
   aac cac gca atg gat tac ggc gtt cag ggc atg aaa gat acg ctt gga      480
   Asn His Ala Met Asp Tyr Gly Val Gln Gly Met Lys Asp Thr Leu Gly
   145                 150                 155                 160
   gaa ttt gcg aag caa aac ctt gat atc gtt gga gcg gga tac agc tta      528
   Glu Phe Ala Lys Gln Asn Leu Asp Ile Val Gly Ala Gly Tyr Ser Leu
                   165                 170                 175
   agt gat gcg aaa aag aaa att tcg tac cag aaa gtc aac ggg gta acg      576
   Ser Asp Ala Lys Lys Lys Ile Ser Tyr Gln Lys Val Asn Gly Val Thr
               180                 185                 190
   att gcg acg ctt ggc ttt acc gat gtg tcc ggg aaa ggt ttc gcg gct      624
   Ile Ala Thr Leu Gly Phe Thr Asp Val Ser Gly Lys Gly Phe Ala Ala
           195                 200                 205
   aaa aag aat acg ccg ggc gtg ctg ccc gca gat cct gaa atc ttc atc      672
   Lys Lys Asn Thr Pro Gly Val Leu Pro Ala Asp Pro Glu Ile Phe Ile
       210                 215                 220
   cct atg att tca gaa gcg aaa aaa cat gcg gac atc gtt gtt gtg cag      720
   Pro Met Ile Ser Glu Ala Lys Lys His Ala Asp Ile Val Val Val Gln
   225                 230                 235                 240
   tca cac tgg ggc caa gag tat gac aat gat cca aac gac cgc cag cgc      768
   Ser His Trp Gly Gln Glu Tyr Asp Asn Asp Pro Asn Asp Arg Gln Arg
                   245                 250                 255
   cag ctt gca aga gcc atg tct gat gcg gga gct gac atc atc gtc ggc      816
   Gln Leu Ala Arg Ala Met Ser Asp Ala Gly Ala Asp Ile Ile Val Gly
               260                 265                 270
   cat cac ccg cac gtc tta gaa ccg att gaa gta tat aac gga acc gtc      864
   His His Pro His Val Leu Glu Pro Ile Glu Val Tyr Asn Gly Thr Val
           275                 280                 285
   att ttc tac agc ctc ggc aac ttt gtc ttt gac caa ggc tgg acg aga      912
   Ile Phe Tyr Ser Leu Gly Asn Phe Val Phe Asp Gln Gly Trp Thr Arg
       290                 295                 300
   aca aga gac agt gca ctg gtt cag tat cac ctg aag aaa aat gga aca      960
   Thr Arg Asp Ser Ala Leu Val Gln Tyr His Leu Lys Lys Asn Gly Thr
   305                 310                 315                 320
   ggc cgc ttt gaa gtg aca ccg atc gac atc cat gaa gcg aca cct gcg     1008
   Gly Arg Phe Glu Val Thr Pro Ile Asp Ile His Glu Ala Thr Pro Ala
                   325                 330                 335
   cct gtg aaa aaa gac agc ctt aaa cag aaa acc att att cgc gaa ctg     1056
   Pro Val Lys Lys Asp Ser Leu Lys Gln Lys Thr Ile Ile Arg Glu Leu
               340                 345                 350
   acg aaa gac tct aat ttc gct tgg aaa gta gaa gac gga aaa ctg acg     1104
   Thr Lys Asp Ser Asn Phe Ala Trp Lys Val Glu Asp Gly Lys Leu Thr
           355                 360                 365
   ttt gat att gat cat agt gac aaa cta aaa tct aaa                     1140
   Phe Asp Ile Asp His Ser Asp Lys Leu Lys Ser Lys
       370                 375                 380
<210> 52
   <211> 380
   <212> PRT
   <213> Bacillus subtilis KSM-366
<400> 52
   Met Lys Lys Glu Leu Ser Phe His Glu Lys Leu Leu Lys Leu Thr Lys
   1               5                   10                  15
   Gln Gln Lys Lys Lys Thr Asn Lys His Val Phe Ile Ala Ile Pro Ile
               20                  25                  30
   Val Phe Val Leu Met Phe Ala Phe Met Trp Ala Gly Lys Ala Glu Thr
           35                  40                  45
   Pro Lys Val Lys Thr Tyr Ser Asp Asp Val Leu Ser Ala Ser Phe Val
       50                  55                  60
   Gly Asp Ile Met Met Gly Arg Tyr Val Glu Lys Val Thr Glu Gln Lys
   65                  70                  75                  80
   Gly Ala Asp Ser Ile Phe Gln Tyr Val Glu Pro Ile Phe Arg Ala Ser
                   85                  90                  95
   Asp Tyr Val Ala Gly Asn Phe Glu Asn Pro Val Thr Tyr Gln Lys Asn
               100                 105                 110
   Tyr Lys Gln Ala Asp Lys Glu Ile His Leu Gln Thr Asn Lys Glu Ser
           115                 120                 125
   Val Lys Val Leu Lys Asp Met Asn Phe Thr Val Leu Asn Ser Ala Asn
       130                 135                 140
   Asn His Ala Met Asp Tyr Gly Val Gln Gly Met Lys Asp Thr Leu Gly
   145                 150                 155                 160
   Glu Phe Ala Lys Gln Asn Leu Asp Ile Val Gly Ala Gly Tyr Ser Leu
                   165                 170                 175
   Ser Asp Ala Lys Lys Lys Ile Ser Tyr Gln Lys Val Asn Gly Val Thr
               180                 185                 190
   Ile Ala Thr Leu Gly Phe Thr Asp Val Ser Gly Lys Gly Phe Ala Ala
           195                 200                 205
   Lys Lys Asn Thr Pro Gly Val Leu Pro Ala Asp Pro Glu Ile Phe Ile
       210                 215                 220
   Pro Met Ile Ser Glu Ala Lys Lys His Ala Asp Ile Val Val Val Gln
   225                 230                 235                 240
   Ser His Trp Gly Gln Glu Tyr Asp Asn Asp Pro Asn Asp Arg Gln Arg
                   245                 250                 255
   Gln Leu Ala Arg Ala Met Ser Asp Ala Gly Ala Asp Ile Ile Val Gly
               260                 265                 270
   His His Pro His Val Leu Glu Pro Ile Glu Val Tyr Asn Gly Thr Val
           275                 280                 285
   Ile Phe Tyr Ser Leu Gly Asn Phe Val Phe Asp Gln Gly Trp Thr Arg
       290                 295                 300
   Thr Arg Asp Ser Ala Leu Val Gln Tyr His Leu Lys Lys Asn Gly Thr
   305                 310                 315                 320
   Gly Arg Phe Glu Val Thr Pro Ile Asp Ile His Glu Ala Thr Pro Ala
                   325                 330                 335
   Pro Val Lys Lys Asp Ser Leu Lys Gln Lys Thr Ile Ile Arg Glu Leu
               340                 345                 350
   Thr Lys Asp Ser Asn Phe Ala Trp Lys Val Glu Asp Gly Lys Leu Thr
           355                 360                 365
   Phe Asp Ile Asp His Ser Asp Lys Leu Lys Ser Lys
       370                 375                 380